# EUROPEAN PATENT SPECIFICATION

(11) **EP 3 458 597 B1**
(45) Date of publication and mention of the grant of the patent: **07.09.2022**
(21) Application number: 17800186.3
(22) Date of filing: 18.05.2017
(51) Int. Cl.: B01L 7/00, B01L 3/00, C12N 9/12, C12Q 1/6844

(54) **QUANTITATIVE REAL TIME PCR AMPLIFICATION USING AN ELECTROWETTING-BASED DEVICE**
QUANTITATIVE ECHTZEIT-PCR-AMPLIFIKATION UNTER VERWENDUNG EINER ELEKTROBENETZUNGSBASIERTEN VORRICHTUNG
AMPLIFICATION PCR QUANTITATIVE EN TEMPS RÉEL À L'AIDE D'UN DISPOSITIF BASÉ SUR L'ÉLECTROMOUILLAGE

(30) Priority: 18.05.2016 US 201662338176 P
(43) Date of publication of application: 27.03.2019
(73) Proprietor: Roche Diagnostics GmbH, 68305 Mannheim (DE); F. Hoffmann-La Roche AG, 4070 Basel (CH)
(72) Inventor: YANG, Jaeyoung, Pleasaton, CA 94566 (US); ASTIER, Yann, Livermore, California 94551 (US); MOK, Janine, Palo Alto, California 94303 (US); SCHLECHT, Ulrich, Sunnyvale, California 94086 (US)
(74) Representative: Burger, Alexander
(86) International application number: PCT/US2017/033374
(87) International publication number: WO 2017/201315

(56) References cited:
- US-A1- 2008 053 205
- US-A1- 2008 274 513
- US-A1- 2008 274 513
- US-A1- 2013 288 254
- US-A1- 2014 030 765
- US-A1- 2014 332 380
- ZHISHAN HUA ET AL: "Multiplexed Real-Time Polymerase Chain Reaction on a Digital Microfluidic Platform", ANALYTICAL CHEMISTRY, vol. 82, no. 6, 15 March 2010 (2010-03-15) , pages 2310-2316, XP055138297, ISSN: 0003-2700, DOI: 10.1021/ac902510u
- SUMIT KALSI ET AL: "Rapid and sensitive detection of antibiotic resistance on a programmable digital microfluidic platform", LAB ON A CHIP, vol. 15, no. 14, 18 June 2015 (2015-06-18) , pages 3065-3075, XP055382821, ISSN: 1473-0197, DOI: 10.1039/C5LC00462D
- PRAKASH R ET AL: "Integrated sample-to-detection chip for nucleic acid test assays", BIOMEDICAL MICRODEVICES, KLUWER, DORDRECHT, NL, vol. 18, no. 3, 11 May 2016 (2016-05-11), pages 1-14, XP035973993, ISSN: 1387-2176, DOI: 10.1007/S10544-016-0069-8 [retrieved on 2016-05-11]

## Description

### FIELD OF THE INVENTION

This invention generally pertains to droplet-based nucleic acid amplification methods. These methods make use of an electrowetting-based device to automate nucleic acid amplification and allow for the quantity of the nucleic acid amplification product to be monitored in real time. Use of an electrowetting-based device subsequently allows for recovery of the desired quantity of amplification product once it has been generated.

### BACKGROUND OF THE INVENTION

Recently, there have been concerted efforts to develop and manufacture microfluidic systems and devices to perform various chemical and biochemical analyses and syntheses, both for preparative and analytical applications. The field of microfluidics generally involves the manipulation of nanoliter or smaller volumes of fluids in devices, generally referred to as microfluidic chips or microfluidic devices, which themselves often feature micron-sized structures. As a result of the precise control possible for manipulations of small volumes of fluids, microfluidic devices allow researchers to perform complex chemical and biological analyses with less volume and in less time than comparable benchtop experimental formats. To date, many microfluidic device designs have been developed that replicate macro-scale experimental techniques while increasing sensitivity and measurements density. With benefits such as a substantial reduction in time, cost, and the space requirements for the devices utilized to conduct a desired analysis or syntheses, microfluidic chips have been designed for such applications as crystallography; PCR; capillary electrophoresis; point-of-care diagnostics; gas chromatography; nucleic acid sequencing; and cell separation, trapping, and subsequent analyses, for example. Additionally, microfluidic devices have the potential to be adapted for use with automated systems, thereby providing the additional benefits of further cost reductions and decreased operator errors because of the reduction in human involvement with the experimental process, and in particular liquid manipulations, e.g., errors associated with pipetting.

In order to promote rapid mixing of reagents and to avoid reagent dispersion, microdroplet-based chips and devices have been developed that compartmentalize reactions into nanoliter-to-picoliter size droplets, often in the form of water-in-oil emulsions. Microdroplets can serve as individual micro-reactors, partitioning customizable blends of reagents into nanoliter-to-picoliter-size reaction volumes. Owing to their low volume and to their use as micro-reactors, microdroplets offer an ideal format for performing reactions in a highly parallelized manner with exquisite assay sensitivity. For example, using a water-in-oil microdroplet-based approach, researchers showed that amplification of a 245 bp Adenovirus product can be detected in 35 minutes at starting template concentrations as low as one template molecule per 167 droplets (0.003 pg/µL) (Kiss et al., Anal. Chem., 2008 Dec 1;80(23):8975-81).

An alternative means for general fluid flow and for microdroplet generation involves the use of electrowetting-based phenomena and electrowetting-based droplet manipulations in a microfluidic device setting. Electrowetting has become established as a lab-on-a-chip technology that is capable of performing complex microfluidic operations such as dispensing, merging, mixing, and splitting microliter-to-nanoliter sized droplets without the need for channel-based structures. Electrowetting-based technology has been implemented in the automation of various molecular biology workflows, including various steps of nucleic acid sequencing sample preparations such as PCR. Electrowetting devices designed to perform the various steps necessary for an assay allows researchers to benefit from the time, cost, reagent, and device footprint savings conferred by microfluidic chips. State of the art technology is disclosed in US 2008/274513 A1 as well as Zhishan et al. (Analytical Chemistry, vol. 82, no. 6, p. 2310-2316, 2010).

### BRIEF SUMMARY OF THE INVENTION

The present invention provides an automated nucleic acid amplification method which comprises the following steps: (a) providing an electrowetting-based device, optionally wherein said electrowetting-based device comprises a biplanar configuration of parallel arrays of electrodes to effect electrowetting-mediated droplet manipulations; (b) providing at least two droplets on said electrowetting-based device, wherein each droplet comprises primers that anneal to a target nucleic acid; and a target nucleic acid; (c) amplifying the target nucleic acid in each said droplet in parallel; (d) quantitating the amplified target nucleic acid in at least one droplet; and (e) after a desired amount of the target nucleic acid has been obtained, recovering at least one droplet using for further analyzing or processing of said at least one droplet, wherein the analyzing or processing is selected from a group consisting of nucleic acid sequencing, next generation sequencing, whole-genome shotgun sequencing, whole exome sequencing, targeted sequencing, amplicon sequencing, mate pair sequencing, RIP-seq/CLIP-seq, ChIP-seq, RNA-seq, transcriptome sequencing, and methyl-seq.

In an embodiment of the invention, said droplets may each comprise a different target nucleic acid. Yet another embodiment of the invention encompasses a method wherein said droplets each may comprise the same target nucleic acid. Yet another embodiment of the invention relates to a method wherein said droplet may comprise a mixture of droplets that contain the same target nucleic acid and different target nucleic acids. Another embodiment of the invention pertains to a method wherein the electrowetting-based device may comprise a biplanar configuration of parallel arrays of electrodes to effect electrowetting-mediated droplet manipulations. Some embodiments relate to a method wherein the electrowetting-based device may comprise a planar configuration of electrodes that effects electrowetting-mediated droplet manipulations. Some embodiments relate to a method wherein the electrowetting-based device may comprise square electrodes, optionally wherein said electrodes are about 5 mm by 5 mm. Some embodiments relate to a method wherein the electrowetting-based device may comprise electrodes, wherein said electrodes are square, triangular, rectangular, circular, trapezoidal, and/or irregularly shaped. Some embodiments relate to a method wherein the electrowetting-based device may comprise electrodes wherein said electrodes may comprise electrode dimensions ranging from about 100 µm by 100 µm to about 10 cm by 10 cm. Some embodiments relate to a method wherein the electrowetting-based device may comprise interdigitated electrodes. Some embodiments relate to a method wherein the electrowetting-based device may comprise electrodes, wherein said electrodes may comprise indium tin oxide ("ITO"), transparent conductive oxides ("TCOs"), conductive polymers, carbon nanotubes ("CNT"), graphene, nanowire meshes and/or ultra thin metal films, e.g., ITO.

Some embodiments relate to a method wherein the electrowetting-based device may comprise droplets, wherein said droplets may comprise a volume ranging from about 1 pL to about 5 mL, e.g., about 12.5 µl. Some embodiments relate to a method wherein the electrowetting-based device may comprise between 1 to about 400 inlet/outlet ports for loading and removal of the same sample or of different samples, and/or said device further comprises between 1 to about 100 inlet/out ports for the introduction and removal of filler fluid(s). Some embodiments relate to a method wherein the electrowetting-based device may comprise inlet/outlet ports wherein the spacing between adjacent ports ranges from about 5 mm to about 500 mm. Additionally, another embodiment pertains to a method wherein the electrowetting-based device may comprise or may be in contact with at least one inductive heating element and at least one detection zone. Another embodiment pertains to a method wherein said heating element may comprise an inductive heating element. A further embodiment generally relates to a method wherein said heating element may comprise a contact heater.

An additional aspect of the invention generally relates to a method wherein the detection zone may detect electrochemical and/or fluorescent signals. Yet another embodiment of the invention relates to a method wherein said detection zone may detect capacitance of a droplet. An additional embodiment of the invention pertains to a method wherein said detection zone may be a fixed location. Another embodiment of the invention relates to a method wherein said detection zone may comprise any location within the electrowetting-based device. Yet another embodiment of the invention generally pertains to a method wherein the method of amplification may comprise hot start PCR. In another embodiment, the invention generally encompasses a method wherein said amplification may comprise isothermal amplification. Another embodiment of the invention relates to a method wherein said amplification may comprise thermocycling. Said thermocycling may comprise temperatures ranging from about 50°C to about 98°C, e.g., about 50°C, about 60°C, about 65°C, about 72°C, about 95°C, or about 98°C. Said thermocycling may comprise times ranging from about 1 s to about 5 min., e.g., about 1 sec, about 5 sec, about 10 sec, about 20 sec, about 30 sec, about 45 sec, about 1 min, and/or about 5 min. Furthermore, said thermocycling may comprise three thermocycle steps, and said three thermocycle steps may be completed in one minute or less.

Yet another embodiment of the invention relates to a method wherein each droplet may further comprise a detection agent. In an additional embodiment, the invention pertains to a method wherein each droplet may contain the same detection agent. Yet another embodiment of the invention generally pertains to a method wherein each droplet may contain a different detection agent. Another embodiment of the invention generally relates to a method wherein the droplets may comprise a labeled subset of droplets wherein each droplet within the subset contains an agent for detecting the target nucleic acid, and an unlabeled subset of droplets wherein each droplet within the subset does not contain said agent for detecting the target nucleic acid. An additional embodiment of the invention generally encompasses a method wherein each droplet within the subset containing a detection agent may comprise a different detection agent. In another embodiment, the invention generally relates to a method wherein each droplet within the subset containing a detection agent may comprise the same detection agent.

An additional aspect of the invention generally relates to a method wherein said further analyzing or processing of said at least one droplet may comprise further analyzing or processing one or more droplets of said unlabeled subset of droplets. In another embodiment, the invention generally pertains to a method wherein each subset of droplets may comprise 1 or more, 2 or more, 10 or more, 100 or more, 1,000 or more, or 10,000 or more droplets. Yet another embodiment of the invention generally relates to a method wherein the agent for detecting the target nucleic acid may comprise a hydrolysis probe, a DNA binding dye, a primer probe, or an analogue of a nucleic acid. In some embodiments, said DNA binding dye may comprise a concentration ranging from about 1 pM to about 1 µM, e.g., about 1 pM, about 10 pM, about 100 pM, about 1 nM, about 10 nM, about 100 nM, or about 1 µM. Some embodiments of the invention generally relate to a method wherein the hydrolysis probe may comprise one or more TaqMan, TaqMan-MGB, and/or Snake primers. Another embodiment of the invention relates to a method wherein said hydrolysis probe may be combined with a fluorophore that effects nucleic acid detection, e.g., wherein said fluorophore may comprise FAM, TET, HEX, VIC, Cy3, Cy5, fluorescein, rhodamine, Oregon green, eosin, Texas red, cyanine, indocarbocyanine, oxacarbocyanine, thiacarbocyanine, merocyanine, hydroxycoumarin, aminocoumarin, methoxycoumarin, Cascade blue, Pacific blue, Pacific orange, Lucifer yellow, R-phycoerthrin, peridinin chlorophyll protein, Fluorx, BODIPY-fluorescein, Cy2, Cy3B, Cy3.5, Cy5.5, Cy7, TRITC, lissamine rhodamine B, or allophycocyanin.

In another embodiment of the invention, said DNA binding dye may comprise ethidium bromide, Sybr Green, Picogreen, Sybr Gold, Syto 9, Syto 13, Syto 16, Sytox blue, chromomycin A3, Os[(bpy)2DPPZ]2+ ("OPD"), BEBO, BETO, BOXTO, Evagreen, propidium iodide, chromomycin, mithramycin, thiazole orange, Cytrak orange, LDS 751, 7-AAD, Sytox green, Sytox orange, TOTO-3, DRAG5, DRAG7, acridine orange, ResoLight, Hoechst 33258, TOTO-1, YOYO-1, YO-PRO-1, TO-PRO-3, or 4',6-diamidino-2-phenylindole ("DAPI"). In some embodiments, said DNA binding dye may comprise OPD, ResoLight, Syto 9, and/or Sybr Green. Another embodiment of the invention generally relates to a method wherein the primer probe may comprise a Scorpion probe, an Amplifuor probe, a Sunrise probe, a Lux probe, a cyclicon probe, or an Angler probe. An additional embodiment of the invention generally relates to a method wherein the hybridization probe may comprise a FRET hybridization probe, a molecular beacon probe, a Hybeacon probe, an MGB probe such as MGB-Pleiades and MGB-Eclipse, a Resonsense probe, or a Yin-Yang probe. Yet another embodiment of the invention generally pertains to a method wherein the analogue of a nucleic acid may comprise a PNA, LNA, ZNA, Plexo primer, or Tiny-Molecular Beacon probe. An additional embodiment of the invention relates to a method wherein said quantitating the amplified target nucleic acid may comprise a detection-based assay, e.g., wherein said detection based assay may comprise an Invader assay, a Nucleic Acid Sequence Based Amplification ("NASBA") assay, and/or capacitive measurement of a droplet. Another embodiment of the invention relates to a method wherein a nucleic acid polymerase may be used to effect amplification of the target nucleic acid. In some embodiments, said polymerase may comprise Kapa HiFi DNA polymerase and/or Kapa Sybr Fast DNA polymerase. Another embodiment of the invention relates to a method wherein the polymerase may be provided from the following: archaea (e.g., *Thermococcus litoralis* (Vent, GenBank: AAA72101), *Pyrococcusfuriosus* (Pfu, GenBank: D12983, BAA02362), *Pyrococcus woesii, Pyrococcus* GB-D (Deep Vent, GenBank: AAA67131), *Thermococcus kodakaraensis* KODI (KOD, GenBank: BD175553, BAA06142; *Thermococcus* sp. strain KOD (Pfx, GenBank: AAE68738), *Thermococcus gorgonarius* (Tgo, Pdb: 4699806), *Sulfolobus solataricus* (GenBank: NC002754, P26811), *Aeropyrum pernix* (GenBank: BAA81109), *Archaeglobus fulgidus* (GenBank: 029753), *Pyrobaculum aerophilum* (GenBank: AAL63952), *Pyrodictium occultum* (GenBank: BAA07579, BAA07580), *Thermococcus* 9 degree Nm (GenBank: AAA88769, Q56366), *Thermococcus fumicolans* (GenBank: CAA93738, P74918), *Thermococcus hydrothermalis* (GenBank: CAC18555), *Thermococcus* sp. GE8 (GenBank: CAC12850), *Thermococcus* sp. JDF-3 (GenBank: AX135456; WO0132887), *Thermococcus* sp. TY (GenBank: CAA73475), *Pyrococcus abyssi* (GenBank: P77916), *Pyrococcus glycovorans* (GenBank: CAC12849), *Pyrococcus horikoshii* (GenBank: NP 143776), *Pyrococcus* sp. GE23 (GenBank: CAA90887), *Pyrococcus* sp. ST700 (GenBank: CAC 12847), *Thermococcus pacificus* (GenBank: AX411312.1), *Thermococcus zilligii* (GenBank: DQ3366890), *Thermococcus aggregans, Thermococcus barossii, Thermococcus celer* (GenBank: DD259850.1), *Thermococcus profundus* (GenBank: E14137), *Thermococcus siculi* (GenBank: DD259857.1), *Thermococcus thioreducens, Thermococcus onnurineus* NA1, *Sulfolobus acidocaldarium, Sulfolobus tokodaii, Pyrobaculum calidifontis, Pyrobaculum islandicum* (GenBank: AAF27815), *Methanococcus jannaschii* (GenBank: Q58295), *Desulforococcus* species TOK, *Desulfurococcus, Pyrolobus, Pyrodictium, Staphylothermus, Vulcanisaetta, Methanococcus* (GenBank: P52025) and other archaeal B polymerases, such as GenBank AAC62712, P956901, BAAA07579)), thermophilic bacteria *Thermus* species (*e.g,,flavus, ruber, thermophilus, lacteus, rubens, aquaticus*)*, Bacillus stearothermophilus, Thermotoga maritima, Methanothermusfervidus,* KOD polymerase, TNA1 polymerase, *Thermococcus* sp. 9 degrees N-7, T4, T7, phi29, *Pyrococcus furiosus, P. abyssi, T. gorgonarius, T. litoralis, T. zilligii, T.* sp. GT, *P.* sp. GB-D, KOD, Pfu, *T. gorgonarius, T. zilligii, T. litoralis* and *Thermococcus* sp. 9N-7 polymerases.

Another embodiment of the invention generally pertains to a method wherein the nucleic acid polymerase may be a modified naturally occurring Type A polymerase. A further embodiment of the invention generally relates to a method wherein the modified Type A polymerase may be selected from any species of the genus *Meiothermus, Thermotoga,* or *Thermomicrobium.* Another embodiment of the invention generally pertains to a method wherein the polymerase may be isolated from any of *Thermus aquaticus* (Taq), *Thermus thermophilus, Thermus caldophilus,* or *Thermus filiformis.* A further embodiment of the invention generally encompasses a method wherein the modified Type A polymerase may be isolated from *Bacillus stearothermophilus, Sphaerobacter thermophilus, Dictoglomus thermophilum,* or *Escherichia coli.* In another embodiment, the invention generally relates to a method wherein the modified Type A polymerase may be a mutant Taq-E507K polymerase. Another embodiment of the invention generally pertains to a method wherein a thermostable polymerase may be used to effect amplification of the target nucleic acid. A further embodiment of the invention generally relates to a method wherein the thermostable polymerase may be selected from the following: *Thermotoga maritima, Thermus aquaticus, Thermus thermophilus, Thermus flavus, Thermus filiformis, Thermus* species Sps17, *Thermus* species Z05, *Thermus caldophilus, Bacillus caldotenax, Thermotoga neopolitana,* and *Thermosipho africanus.* In yet another embodiment, the invention generally relates to a method wherein a modified polymerase may be used to effect amplification of the target nucleic acid, e.g., wherein said modified polymerase may be selected from the following: G46E E678G CS5 DNA polymerase, G46E L329A E678G CS5 DNA polymerase, G46E L329A D640G S671F CS5 DNA polymerase, G46E L329A D640G S671F E678G CS5 DNA polymerase, a G46E E678G CS6 DNA polymerase, Z05 DNA polymerase, ΔZ05 polymerase, AZ05-Gold polymerase, AZ05R polymerase, E615G Taq DNA polymerase, E678G TMA-25 polymerase, and E678G TMA-30 polymerase.

An additional embodiment of the invention generally pertains to a method wherein detection of the detection agent may occur at the end of an amplification cycle. Another embodiment of the invention generally relates to a method wherein detection of the detection agent may occur at any point during the amplification. A further embodiment of the invention generally relates to a method wherein each droplet may comprise on average less than one copy of a nucleic acid sample comprising the target nucleic acid, a single copy of a nucleic acid sample comprising the target nucleic acid, or a concentration of 0.001 pg/µL or more, 0.01 pg/µL or more, 0.1 pg/µL or more, or 1.0 pg/µL or more of a nucleic acid sample comprising the target nucleic acid. In some embodiments, said droplet may comprise a target concentration of amplified material, e.g., a nucleic acid, e.g., a target nucleic acid, ranging from about 1 pM to about 1 mM, e.g., about 1 pM, about 10 pM, about 100 pM, about 1 nM, about 10 nM, about 100 nM, about 1 µM, about 10 µM, about 100 µM, or about 1 mM. In some embodiments, said droplet may comprise a starting concentration of a nucleic acid, e.g., a target nucleic acid, ranging from about 1 pM to about 1 mM, e.g., about 1 pM, about 10 pM, about 100 pM, about 1 nM, about 10 nM, about 100 nM, about 1 µM, about 10 µM, about 100 µM, or about 1 mM. Another embodiment of the invention pertains to a method wherein said target nucleic acid may be derived from a biological sample, e.g., wherein said biological sample may comprise a tumor, lymph node, biopsy, metastases, polyp, cyst, whole blood, saliva, sputum, bacterial cell, virus, lymphatic fluid, serum, plasma, sweat, tear, cerebrospinal fluid, amniotic fluid, seminal fluid, vaginal excretion, serous fluid, synovial fluid, pericardial fluid, peritoneal fluid, pleural fluid, cystic fluid, bile, urine, gastric fluid, intestinal fluid, and/or fecal samples. Another embodiment of the invention generally relates to a method wherein said target nucleic acid may comprise a biomarker. Said biomarker may be selected from: an immune checkpoint inhibitor, CTLA-4, PDL1, PDL2, PD1, B7-H3, B7-H4, BTLA, HVEM, TIM3, GAL9, GITR, LAG3, VISTA, KIR, 2B4, TRPO2, CD160, CGEN-15049, CHK 1, CHK2, A2aR, TL1A, B-7 family ligands, or a ligand of an immune checkpoint inhibitor, or a combination thereof. Additionally, said biomarker may be selected from AKAP4, ALK, APC, AR, BRAF, BRCA1, BRCA2, CCND1, CCND2, CCND3, CD274, CDK4, CDK6, CFB, CFH, CFI, DKK1, DPYD, EDNRB, EGFR, ERBB2, EPSTI1, ESR1, FCRL5, FGFR1, FGFR2, FGFR3, FLT3, FN14, HER2, HER4, HERC5, IDH1, IDH2, IDOl, KIF5B, KIT, KRAS, LGR5, LIV1, LY6E, LYPD3, MACC1, MET, MRD, MSI, MSLN, MUC16, MYC, NaPi3b, NRAS, PDGFRA, PDCD1LG2, RAF1, RNF43, NTRK1, NTSR1, OX40, PIK3CA, RET, ROS1, Septin9, TERT, TFRC, TROP2, TP53, TWEAK, UGT1A1, P13KCA, p53, MAP2K4, ATR, or any other biomarker wherein the expression of which is correlated to a specific cancer.

Another embodiment of the invention generally relates to a method wherein the method may detect a single nucleotide polymorphism. An additional embodiment of the invention generally relates to a method wherein the method may be used for amplicon generation. Another embodiment of the invention generally relates to a method wherein the method may be used for a melting curve analysis. Another embodiment of the invention generally relates to a method wherein the method may be used for target nucleic acid enrichment. A further embodiment of the invention generally relates to a method wherein the method may be used for primer extension target enrichment ("PETE"). An additional embodiment of the invention generally pertains to a method wherein the method may be used to for library amplification. A further embodiment of the invention generally relates to a method wherein the method may be used quantitate the number of adapter-ligated target nucleic acid molecules during library preparation. In another embodiment, the invention generally relates to a method wherein said quantitation of the number of adapter-ligated target nucleic acid molecules may occur (a) after adapter ligation to determine the amount of input material converted to adapter-ligated molecules (conversion rate) and/or the quantity of template used for library amplification; (b) after library amplification, to determine whether a sufficient amount of each library has been generated and/or to ensure equal representation of indexed libraries pooled for target capture or cluster amplification; and/or (c) prior to cluster amplification, to confirm that individual libraries or sample pools are diluted to the optimal concentration for NGS flow cell loading. Additionally, said quantitation of the number of adapter-ligated target nucleic acid molecules may occur after post-ligation cleanup steps (prior to library amplification).

Another embodiment of the invention generally relates to a method wherein after recovering at least one droplet, said further analyzing or processing of said at least one droplet may comprise a nucleic acid sequencing reaction, a next generation sequencing reaction, whole-genome shotgun sequencing, whole exome or targeted sequencing, amplicon sequencing, mate pair sequencing, RIP-seq/CLIP-seq, ChIP-seq, RNA-seq, transcriptome analysis, and/or methyl-seq. In another embodiment, the invention generally relates to a method wherein the droplets may be surrounded by a filler fluid, e.g., wherein said filler fluid may be an oil. In some embodiments, said oil may comprise a transparent oil. In some embodiments, said oil may comprise liquid polymerized siloxane, silicone oil mineral oil, and/or paraffin oil. Another embodiment of the invention generally relates to method wherein the droplets may be surrounded by a gas, e.g., wherein said gas may be air. Yet another embodiment of the invention generally relates to a method wherein the method may be used to avoid overamplification bias. Another embodiment of the invention generally relates to a method wherein the method may be used to produce a representative sample of a population of mutations. In a further embodiment, the invention generally encompasses a method wherein the method may be used to determine the number of amplification cycles necessary to generate the desired concentration of a target nucleic acid. In another embodiment, the invention generally relates to a method wherein the method may be controlled through a computer in communication with the electrowetting-based device. Some embodiments generally relate to a method wherein said method comprises a master mix. In some embodiments, said master mix may comprise a polymerase, dNTP(s), MgCl₂, and/or oligonucleotide primer(s). In some embodiments, said master mix may comprise dNTP(s) at a concentration comprising from about 1 mM to about 100 mM, e.g., about 1 mM, about 10 mM, or about 100 mM; MgCl₂ at a concentration comprising from about 1 mM to about 100 mM, e.g., about 1 mM, about 10 mM, or about 100 mM; and/or a oligonucleotide primer(s) at a concentration comprising from about 1 nM to about 1 mM, e.g., about 1 nM, about 1 µM, or about 1 mM.

Another aspect of the invention generally pertains to a device for amplification of a target nucleic acid, wherein said device may (a) comprise a biplanar configuration of parallel arrays of electrodes to effect electrowetting-mediated droplet manipulations; (b) comprise or be in contact with at least one heating element; and (c) comprise or be in contact with at least one detection zone. In an embodiment of the invention, said heating element may comprise an inductive heating element. Another aspect of the invention generally pertains to a device for amplification of a target nucleic acid, wherein said device may (a) comprise a planar configuration of electrodes to effect electrowetting-mediated droplet manipulations; (b) comprise or be in contact with at least one heating element; and (c) comprise or be in contact with at least one detection zone. In an embodiment of the invention, said heating element may comprise an inductive heating element. In some embodiments, said electrodes may comprise square shapes, optionally about 5 mm by 5 mm. In some embodiments, said electrodes may comprise square, triangular, rectangular, circular, trapezoidal, and/or irregularly shapes. In some embodiments, said electrodes may comprise electrode dimensions ranging from about 100 µm by 100 µm to about 10 cm by 10 cm. In some embodiments, said electrodes may be interdigitated. In some embodiments, said electrodes may comprise indium tin oxide ("ITO"), transparent conductive oxides ("TCOs"), conductive polymers, carbon nanotubes ("CNT"), graphene, nanowire meshes and/or ultra thin metal films, e.g., ITO. In some embodiments, said device may comprise droplets that range in volume from about 1 picoliter to about 5 mL, e.g., about 12.5 µl. In some embodiments, said device may comprise a gap between a top plate and a bottom plate of about 0.5 mm. In some embodiments, said device may comprise a plurality of inlet/outlet ports. In some embodiments, said device may comprise between 1 to about 400 inlet/outlet ports for loading and removal of the same sample or of different samples, and/or said device further comprises between 1 to about 100 inlet/out ports for the introduction and removal of filler fluid(s). In some embodiments, said device may comprise inlet/outlet ports wherein the spacing between adjacent ports ranges from about 5 mm to about 500 mm. In a further embodiment of the invention, said heating element may comprise a contact heater. An additional aspect of the invention relates to an embodiment wherein said amplification comprises thermocycling. Said thermocycling may comprise three thermocycle steps, and said three thermocycle steps may be completed in one minute or less. In another embodiment, said amplification may comprise isothermal amplification. In a further embodiment of the invention, said amplification may comprise hot start PCR. In yet another embodiment of the invention, the detection zone may detect electrochemical and/or fluorescent signals. An additional embodiment of the invention pertains to a detection zone that may detect capacitance of a droplet. In a further embodiment of the invention, said detection zone may be a fixed location. In another embodiment of the invention, said detection zone may comprise any location within the electrowetting-based device. In another embodiment of the invention, the target nucleic acid may be provided on the device within at least three droplets. In a further embodiment of the invention, said droplets may each comprise the same target nucleic acid. In yet another embodiment of the invention, said droplets may comprise a mixture of droplets that contain the same target nucleic acid and different target nucleic acids. In another embodiment of the invention, each droplet may further comprise a detection agent. In an additional embodiment of the invention, each droplet may contain the same detection agent. In another embodiment of the invention, each droplet may contain a different detection agent. Additionally, in another embodiment of the invention, the droplets may comprise a labeled subset of droplets that each contain an agent for detecting the target nucleic acid, and an unlabeled subset of droplets that each do not contain said agent for detecting the target nucleic acid. In an additional embodiment of the invention, each droplet within the subset containing a detection agent may comprise a different detection agent. In another embodiment of the invention, each droplet within the subset containing a detection agent may comprise the same detection agent. In a further embodiment of the invention, each subset of droplets may comprise 1 or more, 2 or more, 10 or more, 100 or more, 1,000 or more, or 10,000 or more droplets.

In an additional embodiment of the invention, the agent for detecting the target nucleic acid may comprise a hydrolysis probe, a DNA binding dye, a primer probe, or an analogue of a nucleic acid. In another embodiment of the invention, the hydrolysis probe may comprise one or more TaqMan, TaqMan-MGB, and/or Snake primers. In a further embodiment of the invention, said hydrolysis probe may be combined with a fluorophore that effects nucleic acid detection, e.g., wherein said fluorophore may comprise FAM, TET, HEX, VIC, Cy3, Cy5, fluorescein, rhodamine, Oregon green, eosin, Texas red, cyanine, indocarbocyanine, oxacarbocyanine, thiacarbocyanine, merocyanine, hydroxycoumarin, aminocoumarin, methoxycoumarin, Cascade blue, Pacific blue, Pacific orange, Lucifer yellow, R-phycoerthrin, peridinin chlorophyll protein, Fluorx, BODIPY-fluorescein, Cy2, Cy3B, Cy3.5, Cy5.5, Cy7, TRITC, lissamine rhodamine B, or allophycocyanin. In yet another embodiment of the invention, the DNA binding dye may comprise ethidium bromide, Sybr Green, Picogreen, Sybr Gold, Syto 9, Syto 13, Syto 16, Sytox blue, chromomycin A3, Os[(bpy)2DPPZ]2+ ("OPD"), BEBO, BETO, BOXTO, Evagreen, propidium iodide, chromomycin, mithramycin, thiazole orange, Cytrak orange, LDS 751, 7-AAD, Sytox green, Sytox orange, TOTO-3, DRAG5, DRAG7, acridine orange, ResoLight, Hoechst 33258, TOTO-1, YOYO-1, YO-PRO-1, TO-PRO-3, or 4',6-diamidino-2-phenylindole ("DAPI"). In yet another embodiment of the invention, the primer probe may comprise a Scorpion probe, an Amplifuor probe, a Sunrise probe, a Lux probe, a cyclicon probe, or an Angler probe. In another embodiment of the invention, the hybridization probe may comprise a FRET hybridization probe, a molecular beacon probe, a Hybeacon probe, an MGB probe such as MGB-Pleiades and MGB-Eclipse, a Resonsense probe, or a Yin-Yang probe. In a further embodiment of the invention, the analogue of a nucleic acid may comprise a PNA, LNA, ZNA, Plexo primer, or Tiny-Molecular Beacon probe. Moreover, in another embodiment of the invention, the detection agent may comprise a detection-based assay, e.g., wherein said detection-based assay may comprise an Invader assay, a Nucleic Acid Sequence Based Amplification ("NASBA") assay, and/or capacitive measurement of a droplet.

In an additional embodiment of the invention, a nucleic acid polymerase is used to effect amplification of the target nucleic acid. In another embodiment of the invention, the polymerase may be provided from the following: archaea (e.g., *Thermococcus litoralis* (Vent, GenBank: AAA72101), *Pyrococcusfuriosus* (Pfu, GenBank: D12983, BAA02362), *Pyrococcus woesii, Pyrococcus* GB-D (Deep Vent, GenBank: AAA67131), *Thermococcus kodakaraensis* KODI (KOD, GenBank: BD175553, BAA06142; *Thermococcus* sp. strain KOD (Pfx, GenBank: AAE68738)), *Thermococcus gorgonarius* (Tgo, Pdb: 4699806), *Sulfolobus solataricus* (GenBank: NC002754, P26811), *Aeropyrum pernix* (GenBank: BAA81109), *Archaeglobus fulgidus* (GenBank: 029753), *Pyrobaculum aerophilum* (GenBank: AAL63952), *Pyrodictium occultum* (GenBank: BAA07579, BAA07580), *Thermococcus* 9 degree Nm (GenBank: AAA88769, Q56366), *Thermococcus fumicolans* (GenBank: CAA93738, P74918), *Thermococcus hydrothermalis* (GenBank: CAC18555), *Thermococcus* sp. GE8 (GenBank: CAC12850), *Thermococcus* sp. JDF-3 (GenBank: AX135456; WO0132887), *Thermococcus* sp. TY (GenBank: CAA73475), *Pyrococcus abyssi* (GenBank: P77916), *Pyrococcus glycovorans* (GenBank: CAC12849), *Pyrococcus horikoshii* (GenBank: NP 143776), *Pyrococcus* sp. GE23 (GenBank: CAA90887), *Pyrococcus* sp. ST700 (GenBank: CAC 12847), *Thermococcus pacificus* (GenBank: AX411312.1), *Thermococcus zilligii* (GenBank: DQ3366890), *Thermococcus aggregans, Thermococcus barossii, Thermococcus celer* (GenBank: DD259850.1), *Thermococcus profundus* (GenBank: E14137), *Thermococcus siculi* (GenBank: DD259857.1), *Thermococcus thioreducens, Thermococcus onnurineus* NA1, *Sulfolobus acidocaldarium, Sulfolobus tokodaii, Pyrobaculum calidifontis, Pyrobaculum islandicum* (GenBank: AAF27815), *Methanococcus jannaschii* (GenBank: Q58295), *Desulforococcus* species TOK, *Desulfurococcus, Pyrolobus, Pyrodictium, Staphylothermus, Vulcanisaetta, Methanococcus* (GenBank: P52025) and other archaeal B polymerases, such as GenBank AAC62712, P956901, BAAA07579)), thermophilic bacteria *Thermus* species (*e.g,,flavus, ruber, thermophilus, lacteus, rubens, aquaticus), Bacillus stearothermophilus, Thermotoga maritima, Methanothermus fervidus,* KOD polymerase, TNA1 polymerase, *Thermococcus* sp. 9 degrees N-7, T4, T7, phi29, *Pyrococcus furiosus, P. abyssi, T. gorgonarius, T. litoralis, T. zilligii, T.* sp. GT, *P.* sp. GB-D, KOD, Pfu, *T. gorgonarius, T. zilligii, T. litoralis* and *Thermococcus* sp. 9N-7 polymerases. In another embodiment of the invention, the nucleic acid polymerase may be a modified naturally occurring Type A polymerase. In a further embodiment of the invention, wherein the modified Type A polymerase may be selected from any species of the genus *Meiothermus, Thermotoga,* or *Thermomicrobium.* In another embodiment of the invention, the modified Type A polymerase may be isolated from any of *Thermus aquaticus* (Taq), *Thermus thermophilus, Thermus caldophilus,* or *Thermus filiformis.* In an additional embodiment of the invention, the modified Type A polymerase may be isolated from *Bacillus stearothermophilus, Sphaerobacter thermophilus, Dictoglomus thermophilum,* or *Escherichia coli.* In yet another embodiment of the invention, the modified Type A polymerase may be a mutant Taq-E507K polymerase. In another embodiment of the invention, a thermostable polymerase may be used to effect amplification of the target nucleic acid. In yet another embodiment of the invention, the thermostable polymerase may be selected from the following: *Thermotoga maritima, Thermus aquaticus, Thermus thermophilus, Thermus flavus, Thermus filiformis, Thermus* species Sps17, *Thermus* species Z05, *Thermus caldophilus, Bacillus caldotenax, Thermotoga neopolitana,* and *Thermosipho africanus.* In a further embodiment of the invention, a modified polymerase may be used to effect amplification of the target nucleic acid. In another embodiment of the invention, the modified polymerase may be selected from the following: include G46E E678G CS5 DNA polymerase, G46E L329A E678G CS5 DNA polymerase, G46E L329A D640G S671F CS5 DNA polymerase, G46E L329A D640G S671F E678G CS5 DNA polymerase, a G46E E678G CS6 DNA polymerase, Z05 DNA polymerase, ΔZ05 polymerase, AZ05-Gold polymerase, AZ05R polymerase, E615G Taq DNA polymerase, E678G TMA-25 polymerase, and E678G TMA-30 polymerase.

In yet another embodiment of the invention, the droplets may be thermocycled in parallel. In a further embodiment of the invention, the agent may be used to quantitate the amount of target nucleic acid that has been amplified. In another embodiment of the invention, detection of the detection agent may occur at the end of an amplification cycle. In another embodiment of the invention, detection of the detection agent may occur at any point during the amplification. In another embodiment of the invention, each droplet may comprise on average less than one copy of a nucleic acid sample comprising the target nucleic acid. In a further embodiment of the invention, each droplet may comprise a single copy of a nucleic acid sample comprising the target nucleic acid, or each droplet may comprise a concentration of 0.001 pg/µL or more, 0.01 pg/µL or more, 0.1 pg/µL or more, or 1.0 pg/µL or more of a nucleic acid sample comprising the target nucleic acid. In another embodiment of the invention, said target nucleic acid may be derived from a biological sample, e.g., wherein the biological sample may comprise a tumor, lymph node, biopsy, metastases, polyp, cyst, whole blood, saliva, sputum, bacterial cell, virus, lymphatic fluid, serum, plasma, sweat, tear, cerebrospinal fluid, amniotic fluid, seminal fluid, vaginal excretion, serous fluid, synovial fluid, pericardial fluid, peritoneal fluid, pleural fluid, cystic fluid, bile, urine, gastric fluid, intestinal fluid, and/or fecal samples. In another embodiment of the invention, said target nucleic acid may comprise a biomarker. Said biomarker may be selected from: an immune checkpoint inhibitor, CTLA-4, PDL1, PDL2, PD1, B7-H3, B7-H4, BTLA, HVEM, TIM3, GAL9, GITR, LAG3, VISTA, KIR, 2B4, TRPO2, CD160, CGEN-15049, CHK 1, CHK2, A2aR, TL1A, B-7 family ligands, or a ligand of an immune checkpoint inhibitor, or a combination thereof. Additionally, said biomarker may be selected from AKAP4, ALK, APC, AR, BRAF, BRCA1, BRCA2, CCND1, CCND2, CCND3, CD274, CDK4, CDK6, CFB, CFH, CFI, DKK1, DPYD, EDNRB, EGFR, ERBB2, EPSTI1, ESR1, FCRL5, FGFR1, FGFR2, FGFR3, FLT3, FN14, HER2, HER4, HERC5, IDH1, IDH2, IDOl, KIF5B, KIT, KRAS, LGR5, LIV1, LY6E, LYPD3, MACC1, MET, MRD, MSI, MSLN, MUC16, MYC, NaPi3b, NRAS, PDGFRA, PDCD1LG2, RAF1, RNF43, NTRK1, NTSR1, OX40, PIK3CA, RET, ROS1,Septin 9,TERT,TFRC,TROP2,TP53, TWEAK, UGT1A1, P13KCA, p53, MAP2K4, ATR, or any other biomarker wherein the expression of which is correlated to a specific cancer.

In another embodiment of the invention, the device may detect a single nucleotide polymorphism. In yet another embodiment of the invention, the device may effect amplicon generation. In an additional embodiment of the invention, the device may effect a melting curve analysis. In yet another embodiment of the invention, the device may effect target nucleic acid enrichment. In yet another embodiment of the invention, the device may effect PETE. In an additional embodiment of the invention, the device may effect library amplification. In a further embodiment of the invention, the device may quantitate the number of adapter-ligated target nucleic acid molecules during library preparation. For example, said quantitation may occur (a) after adapter ligation to determine the amount of input material converted to adapter-ligated molecules (conversion rate) and/or the quantity of template used for library amplification; (b) after library amplification, to determine whether a sufficient amount of each library has been generated and/or to ensure equal representation of indexed libraries pooled for target capture or cluster amplification; and/or (c) prior to cluster amplification, to confirm that individual libraries or sample pools are diluted to the optimal concentration for NGS flow cell loading. Also, said quantitation may occur after post-ligation cleanup steps (prior to library amplification). In yet another embodiment of the invention, after a desired amount of the target nucleic acid has been obtained, at least one droplet may be recovered from said device prior to further analysis or processing of said droplet. For example, said further analyzing or processing of said at least one droplet may comprise a nucleic acid sequencing reaction, a next generation sequencing reaction, whole-genome shotgun sequencing, whole exome or targeted sequencing, amplicon sequencing, mate pair sequencing, RIP-seq/CLIP-seq, ChIP-seq, RNA-seq, transcriptome analysis, and/or methyl-seq.

In another embodiment of the invention, the droplets may be surrounded by a filler fluid, e.g., wherein said filler fluid may be an oil. In yet another embodiment of the invention, the droplets may be surrounded by a gas, e.g., wherein said gas is air. In a further embodiment of the invention, the device may avoid overamplification bias. In another embodiment of the invention, the device may produce a representative sample of a population of mutations. In a further embodiment of the invention, the device may determine the number of amplification cycles necessary to generate the desired concentration of target nucleic acid. In yet another embodiment of the invention, the device may be controlled through a computer in communication with the electrowetting-based device.

Another aspect of the invention generally relates to a system for automated amplification of a target nucleic acid which may comprise: (a) an electrowetting-based device; (b) at least one heating element that comprises or is in contact with the electrowetting-based device; (c) at least one detection zone that comprises or is in contact with the electrowetting-based device. In an embodiment of the invention, said heating element may comprise an inductive heating element. In a further embodiment of the invention, said heating element may comprise a contact heater. An additional aspect of the invention relates to an embodiment wherein said amplification comprises thermocycling. Said thermocycling may comprise three thermocycle steps, and said three thermocycle steps may be completed in one minute or less. In another embodiment, said amplification may comprise isothermal amplification. In a further embodiment of the invention said amplification may comprise hot start PCR. In yet another embodiment of the invention, the detection zone may detect electrochemical and/or fluorescent signals. An additional embodiment of the invention pertains to a detection zone that may detect capacitance of a droplet. In a further embodiment of the invention, said detection zone may be a fixed location. In another embodiment of the invention, said detection zone may comprise any location within the system. In another embodiment of the invention, the target nucleic acid may be provided on the system within at least three droplets. In a further embodiment of the invention, said droplets may each comprise the same target nucleic acid. In yet another embodiment of the invention, said droplets may comprise a mixture of droplets that contain the same target nucleic acid and different target nucleic acids. In another embodiment of the invention, each droplet may further comprise a detection agent. In an additional embodiment of the invention, each droplet may contain the same detection agent. In another embodiment of the invention, each droplet may contain a different detection agent. Additionally, in another embodiment of the invention, the droplets may comprise a labeled subset of droplets that each contain an agent for detecting the target nucleic acid, and an unlabeled subset of droplets that each do not contain said agent for detecting the target nucleic acid. In an additional embodiment of the invention, each droplet within the subset containing a detection agent may comprise a different detection agent. In another embodiment of the invention, each droplet within the subset containing a detection agent may comprise the same detection agent. In a further embodiment of the invention, each subset of droplets may comprise 1 or more, 2 or more, 10 or more, 100 or more, 1,000 or more, or 10,000 or more droplets.

In an additional embodiment of the invention, the agent for detecting the target nucleic acid may comprise a hydrolysis probe, a DNA binding dye, a primer probe, or an analogue of a nucleic acid. In another embodiment of the invention, the hydrolysis probe may comprise one or more TaqMan, TaqMan-MGB, and/or Snake primers. In a further embodiment of the invention, said hydrolysis probe may be combined with a fluorophore that effects nucleic acid detection, e.g., wherein said fluorophore may comprise FAM, TET, HEX, VIC, Cy3, Cy5, fluorescein, rhodamine, Oregon green, eosin, Texas red, cyanine, indocarbocyanine, oxacarbocyanine, thiacarbocyanine, merocyanine, hydroxycoumarin, aminocoumarin, methoxycoumarin, Cascade blue, Pacific blue, Pacific orange, Lucifer yellow, R-phycoerthrin, peridinin chlorophyll protein, Fluorx, BODIPY-fluorescein, Cy2, Cy3B, Cy3.5, Cy5.5, Cy7, TRITC, lissamine rhodamine B, or allophycocyanin. In yet another embodiment of the invention, the DNA binding dye may comprise ethidium bromide, Sybr Green, Picogreen, Sybr Gold, Syto 9, Syto 13, Syto 16, Sytox blue, chromomycin A3, Os[(bpy)2DPPZ]2+, BEBO, BETO, BOXTO, Evagreen, propidium iodide, chromomycin, mithramycin, thiazole orange, Cytrak orange, LDS 751, 7-AAD, Sytox green, Sytox orange, TOTO-3, DRAG5, DRAG7, acridine orange, ResoLight, Hoechst 33258, TOTO-1, YOYO-1, YO-PRO-1, TO-PRO-3, or 4',6-diamidino-2-phenylindole ("DAPI"). In yet another embodiment of the invention, the primer probe may comprise a Scorpion probe, an Amplifuor probe, a Sunrise probe, a Lux probe, a cyclicon probe, or an Angler probe. In another embodiment of the invention, the hybridization probe may comprise a FRET hybridization probe, a molecular beacon probe, a Hybeacon probe, an MGB probe such as MGB-Pleiades and MGB-Eclipse, a Resonsense probe, or a Yin-Yang probe. In a further embodiment of the invention, the analogue of a nucleic acid may comprise a PNA, LNA, ZNA, Plexo primer, or Tiny-Molecular Beacon probe. Moreover, in another embodiment of the invention, the detection agent may comprise a detection-based assay, e.g., wherein said detection-based assay may comprise an Invader assay, a Nucleic Acid Sequence Based Amplification ("NASBA") assay, and/or capacitive measurement of a droplet.

In an additional embodiment of the invention, a nucleic acid polymerase is used to effect amplification of the target nucleic acid. In another embodiment of the invention, the polymerase may be provided from the following: archaea (e.g., *Thermococcus litoralis* (Vent, GenBank: AAA72101), *Pyrococcusfuriosus* (Pfu, GenBank: D12983, BAA02362), *Pyrococcus woesii, Pyrococcus* GB-D (Deep Vent, GenBank: AAA67131), *Thermococcus kodakaraensis* KODI (KOD, GenBank: BD175553, BAA06142; *Thermococcus* sp. strain KOD (Pfx, GenBank: AAE68738)), *Thermococcus gorgonarius* (Tgo, Pdb: 4699806), *Sulfolobus solataricus* (GenBank: NC002754, P26811), *Aeropyrum pernix* (GenBank: BAA81109), *Archaeglobus fulgidus* (GenBank: 029753), *Pyrobaculum aerophilum* (GenBank: AAL63952), *Pyrodictium occultum* (GenBank: BAA07579, BAA07580), *Thermococcus* 9 degree Nm (GenBank: AAA88769, Q56366), *Thermococcus fumicolans* (GenBank: CAA93738, P74918), *Thermococcus hydrothermalis* (GenBank: CAC18555), *Thermococcus* sp. GE8 (GenBank: CAC12850), *Thermococcus* sp. JDF-3 (GenBank: AX135456; WO0132887), *Thermococcus* sp. TY (GenBank: CAA73475), *Pyrococcus abyssi* (GenBank: P77916), *Pyrococcus glycovorans* (GenBank: CAC12849), *Pyrococcus horikoshii* (GenBank: NP 143776), *Pyrococcus* sp. GE23 (GenBank: CAA90887), *Pyrococcus* sp. ST700 (GenBank: CAC 12847), *Thermococcus pacificus* (GenBank: AX411312.1), *Thermococcus zilligii* (GenBank: DQ3366890), *Thermococcus aggregans, Thermococcus barossii, Thermococcus celer* (GenBank: DD259850.1), *Thermococcus profundus* (GenBank: E14137), *Thermococcus siculi* (GenBank: DD259857.1), *Thermococcus thioreducens, Thermococcus onnurineus* NA1, *Sulfolobus acidocaldarium, Sulfolobus tokodaii, Pyrobaculum calidifontis, Pyrobaculum islandicum* (GenBank: AAF27815), *Methanococcus jannaschii* (GenBank: Q58295), *Desulforococcus* species TOK, *Desulfurococcus, Pyrolobus, Pyrodictium, Staphylothermus, Vulcanisaetta, Methanococcus* (GenBank: P52025) and other archaeal B polymerases, such as GenBank AAC62712, P956901, BAAA07579)), thermophilic bacteria *Thermus* species (*e.g,,flavus, ruber, thermophilus, lacteus, rubens, aquaticus), Bacillus stearothermophilus, Thermotoga maritima, Methanothermus fervidus,* KOD polymerase, TNA1 polymerase, *Thermococcus* sp. 9 degrees N-7, T4, T7, phi29, *Pyrococcus furiosus, P. abyssi, T. gorgonarius, T. litoralis, T. zilligii, T.* sp. GT, *P.* sp. GB-D, KOD, Pfu, *T. gorgonarius, T. zilligii, T. litoralis* and *Thermococcus* sp. 9N-7 polymerases. In another embodiment of the invention, the nucleic acid polymerase may be a modified naturally occurring Type A polymerase. In a further embodiment of the invention, wherein the modified Type A polymerase may be selected from any species of the genus *Meiothermus, Thermotoga,* or *Thermomicrobium.* In another embodiment of the invention, the modified Type A polymerase may be isolated from any of *Thermus aquaticus* (Taq), *Thermus thermophilus, Thermus caldophilus,* or *Thermus filiformis.* In an additional embodiment of the invention, the modified Type A polymerase may be isolated from *Bacillus stearothermophilus, Sphaerobacter thermophilus, Dictoglomus thermophilum,* or *Escherichia coli.* In yet another embodiment of the invention, the modified Type A polymerase may be a mutant Taq-E507K polymerase. In another embodiment of the invention, a thermostable polymerase may be used to effect amplification of the target nucleic acid. In yet another embodiment of the invention, the thermostable polymerase may be selected from the following: *Thermotoga maritima, Thermus aquaticus, Thermus thermophilus, Thermus flavus, Thermus filiformis, Thermus* species Sps17, *Thermus* species Z05, *Thermus caldophilus, Bacillus caldotenax, Thermotoga neopolitana,* and *Thermosipho africanus.* In a further embodiment of the invention, a modified polymerase may be used to effect amplification of the target nucleic acid. In another embodiment of the invention, the modified polymerase may be selected from the following: include G46E E678G CS5 DNA polymerase, G46E L329A E678G CS5 DNA polymerase, G46E L329A D640G S671F CS5 DNA polymerase, G46E L329A D640G S671F E678G CS5 DNA polymerase, a G46E E678G CS6 DNA polymerase, Z05 DNA polymerase, ΔZ05 polymerase, AZ05-Gold polymerase, AZ05R polymerase, E615G Taq DNA polymerase, E678G TMA-25 polymerase, and E678G TMA-30 polymerase.

In yet another embodiment of the invention, the droplets may be thermocycled in parallel. In a further embodiment of the invention, the agent may be used to quantitate the amount of target nucleic acid that has been amplified. In another embodiment of the invention, detection of the detection agent may occur at the end of an amplification cycle. In another embodiment of the invention, detection of the detection agent may occur at any point during the amplification. In another embodiment of the invention, each droplet may comprise on average less than one copy of a nucleic acid sample comprising the target nucleic acid. In a further embodiment of the invention, each droplet may comprise a single copy of a nucleic acid sample comprising the target nucleic acid, or each droplet may comprise a concentration of 0.001 pg/µL or more, 0.01 pg/µL or more, 0.1 pg/µL or more, or 1.0 pg/µL or more of a nucleic acid sample comprising the target nucleic acid. In another embodiment of the invention, said target nucleic acid may be derived from a biological sample, e.g., wherein the biological sample may comprise a tumor, lymph node, biopsy, metastases, polyp, cyst, whole blood, saliva, sputum, bacterial cell, virus, lymphatic fluid, serum, plasma, sweat, tear, cerebrospinal fluid, amniotic fluid, seminal fluid, vaginal excretion, serous fluid, synovial fluid, pericardial fluid, peritoneal fluid, pleural fluid, cystic fluid, bile, urine, gastric fluid, intestinal fluid, and/or fecal samples. In another embodiment of the invention, said target nucleic acid may comprise a biomarker. Said biomarker may be selected from: an immune checkpoint inhibitor, CTLA-4, PDL1, PDL2, PD1, B7-H3, B7-H4, BTLA, HVEM, TIM3, GAL9, GITR, LAG3, VISTA, KIR, 2B4, TRPO2, CD160, CGEN-15049, CHK 1, CHK2, A2aR, TL1A, B-7 family ligands, or a ligand of an immune checkpoint inhibitor, or a combination thereof. Additionally, said biomarker may be selected from AKAP4, ALK, APC, AR, BRAF, BRCA1, BRCA2, CCND1, CCND2, CCND3, CD274, CDK4, CDK6, CFB, CFH, CFI, DKK1, DPYD, EDNRB, EGFR, ERBB2, EPSTI1, ESR1, FCRL5, FGFR1, FGFR2, FGFR3, FLT3, FN14, HER2, HER4, HERC5, IDH1, IDH2, IDOl, KIF5B, KIT, KRAS, LGR5, LIV1, LY6E, LYPD3, MACC1, MET, MRD, MSI, MSLN, MUC16, MYC, NaPi3b, NRAS, PDGFRA, PDCD1LG2, RAF1, RNF43, NTRK1, NTSR1, OX40, PIK3CA, RET, ROS1,Septin 9,TERT,TFRC,TROP2,TP53, TWEAK, UGT1A1, P13KCA, p53, MAP2K4, ATR, or any other biomarker wherein the expression of which is correlated to a specific cancer.

In another embodiment of the invention, the system may detect a single nucleotide polymorphism. In yet another embodiment of the invention, the system may effect amplicon generation. In an additional embodiment of the invention, the system may effect a melting curve analysis. In yet another embodiment of the invention, the system may effect target nucleic acid enrichment. In yet another embodiment of the invention, the system may effect PETE. In an additional embodiment of the invention, the system may effect library amplification. In a further embodiment of the invention, the system may quantitate the number of adapter-ligated target nucleic acid molecules during library preparation. For example, said quantitation may occur (a) after adapter ligation to determine the amount of input material converted to adapter-ligated molecules (conversion rate) and/or the quantity of template used for library amplification; (b) after library amplification, to determine whether a sufficient amount of each library has been generated and/or to ensure equal representation of indexed libraries pooled for target capture or cluster amplification; and/or (c) prior to cluster amplification, to confirm that individual libraries or sample pools are diluted to the optimal concentration for NGS flow cell loading. Also, said quantitation may occur after post-ligation cleanup steps (prior to library amplification). In yet another embodiment of the invention, after a desired amount of the target nucleic acid has been obtained, at least one droplet may be recovered from said system prior to further analysis or processing of said droplet. For example, said further analyzing or processing of said at least one droplet may comprise a nucleic acid sequencing reaction, a next generation sequencing reaction, whole-genome shotgun sequencing, whole exome or targeted sequencing, amplicon sequencing, mate pair sequencing, RIP-seq/CLIP-seq, ChIP-seq, RNA-seq, transcriptome analysis, and/or methyl-seq.

In another embodiment of the invention, the droplets may be surrounded by a filler fluid, e.g., wherein said filler fluid may be an oil. In yet another embodiment of the invention, the droplets may be surrounded by a gas, e.g., wherein said gas is air. In a further embodiment of the invention, the system may avoid overamplification bias. In another embodiment of the invention, the system may produce a representative sample of a population of mutations. In a further embodiment of the invention, the system may determine the number of amplification cycles necessary to generate the desired concentration of target nucleic acid. In yet another embodiment of the invention, the system may be controlled through a computer in communication with the system.

Another embodiment of the invention generally relates to an automated amplification method which may comprise (a) providing an electrowetting-based device with a biplanar configuration of parallel arrays of electrodes to effect electrowetting-mediated droplet manipulations, and further wherein said device comprises at least one inductive heating element and at least one detection zone; (b) providing on said device droplets comprising a target nucleic acid, wherein said droplets comprise a subset of droplets that contains an agent for target nucleic acid detection comprising a DNA binding dye and a subset of droplets that does not contain said agent for target nucleic acid detection; (c) thermocycling said droplets in parallel, wherein said thermocycling amplifies said target nucleic acid; (d) quantitating the amplified target nucleic acid in said subset of droplets containing said agent through detection of the DNA binding dye; and (e) after a desired amount of said target nucleic acid has been obtained in said subset droplets containing an agent, recovering at least one droplet from said subset of droplets not containing an agent for further analyzing or processing.

Yet another embodiment of the invention generally pertains to an automated amplification method which may comprise (a) providing an electrowetting-based device with a biplanar configuration of parallel arrays of electrodes to effect electrowetting-mediated droplet manipulations, and further wherein said device contains at least one inductive heating element and at least one detection zone; (b) providing on said device droplets comprising a target nucleic acid, wherein said droplets comprise a subset of droplets that contains an agent for target nucleic acid detection comprising a DNA binding dye and a subset of droplets that does not contain said agent for target nucleic acid detection; (c) amplifying the target nucleic acid in each said droplet in parallel; (d) quantitating the amplified target nucleic acid in said subset of droplets containing said agent through detection of the DNA binding dye; and (e) after a desired amount of said target nucleic acid has been obtained in said subset of droplets containing an agent, recovering at least one droplet from said subset of droplets not containing an agent for further analyzing or processing.

An additional embodiment of the invention generally relates to an automated amplification method which may comprise (a) providing an electrowetting-based device with a biplanar configuration of parallel arrays of electrodes to effect electrowetting-mediated droplet manipulations, and further wherein said device contains at least one inductive heating element and at least one detection zone; (b) providing on said device droplets comprising a target nucleic acid, wherein said droplets comprise a subset of droplets that contains an agent for target nucleic acid detection and a subset of droplets that does not contain said agent for target nucleic acid detection; (c) amplifying the target nucleic acid in each said droplet in parallel; (d) quantitating the amplified target nucleic acid in said subset of droplets containing said agent through detection of said agent; and (e) after a desired amount of said target nucleic acid has been obtained in said subset of droplets containing an agent, recovering at least one droplet from said subset of droplets not containing an agent for further analyzing or processing.

Yet another embodiment of the invention generally encompasses an automated amplification method which may comprise (a) providing an electrowetting-based device with a biplanar configuration of parallel arrays of electrodes to effect electrowetting-mediated droplet manipulations, and further wherein said device comprises at least one inductive heating element and at least one detection zone; (b) providing on said device at least 2 droplets which each comprise a different target nucleic acid, and further wherein said droplets comprise an agent for target nucleic acid detection comprising a DNA binding dye; (c) thermocycling said droplets in parallel, wherein said thermocycling amplifies said target nucleic acid; (d) quantitating the amplified target nucleic acid in said droplets through detection of the DNA binding dye; and (e) after a desired amount of said target nucleic acid has been obtained in said droplets, recovering at least one droplet for further analyzing or processing.

Another embodiment of the invention generally relates to an automated amplification method which may comprises (a) providing an electrowetting-based device with a biplanar configuration of parallel arrays of electrodes to effect electrowetting-mediated droplet manipulations, and further wherein said device contains at least one inductive heating element and at least one detection zone; (b) providing on said device at least 2 droplets which each comprise a different target nucleic acid, and further wherein said droplets comprise an agent for target nucleic acid detection comprising a DNA binding dye; (c) amplifying the target nucleic acid in each said droplet in parallel; (d) quantitating the amplified target nucleic acid in said subset of droplets containing said agent through detection of the DNA binding dye; and (e) after a desired amount of said target nucleic acid has been obtained in said droplets, recovering at least one droplet for further analyzing or processing.

Yet another embodiment of the invention generally pertains to an automated PCR amplification method which may comprise (a) providing an electrowetting-based device with a biplanar configuration of parallel arrays of electrodes to effect electrowetting-mediated droplet manipulations, and further wherein said device contains at least one inductive heating element and at least one detection zone; (b) providing on said device at least 2 droplets which each comprise a different target nucleic acid, and further wherein said droplets comprise an agent for target nucleic acid detection; (c) amplifying the target nucleic acid in each said droplet in parallel; (d) quantitating the amplified target nucleic acid in said subset of droplets containing said agent through detection of said agent; and (e) after a desired amount of said target nucleic acid has been obtained in said droplets, recovering at least one droplet for further analyzing or processing.

Yet another embodiment of the invention generally pertains to an automated amplification method which comprises (a) providing an electrowetting-based device for effecting electrowetting-mediated droplet manipulations, and further wherein said device comprises at least one inductive heating element and at least one detection zone; (b) providing on said device droplets comprising a target nucleic acid, wherein said droplets comprise a subset of droplets that contains an agent for target nucleic acid detection comprising a DNA binding dye and a subset of droplets that does not contain said agent for target nucleic acid detection; (c) thermocycling said droplets in parallel, wherein said thermocycling amplifies said target nucleic acid; (d) quantitating the amplified target nucleic acid in said subset of droplets containing said agent through detection of the DNA binding dye; and (e) after a desired amount of said target nucleic acid has been obtained in said subset droplets containing an agent, recovering at least one droplet from said subset of droplets not containing an agent for further analyzing or processing. Yet another aspect of the invention generally pertains to an automated amplification method which comprises (a) providing an electrowetting-based device for effecting electrowetting-mediated droplet manipulations, and further wherein said device contains at least one inductive heating element and at least one detection zone; (b) providing on said device droplets comprising a target nucleic acid, wherein said droplets comprise a subset of droplets that contains an agent for target nucleic acid detection comprising a DNA binding dye and a subset of droplets that does not contain said agent for target nucleic acid detection; (c) amplifying the target nucleic acid in each said droplet in parallel; (d) quantitating the amplified target nucleic acid in said subset of droplets containing said agent through detection of the DNA binding dye; and (e) after a desired amount of said target nucleic acid has been obtained in said subset of droplets containing an agent, recovering at least one droplet from said subset of droplets not containing an agent for further analyzing or processing.

Yet another embodiment of the invention generally pertains to an automated amplification method which comprises (a) providing an electrowetting-based device for effecting electrowetting-mediated droplet manipulations, and further wherein said device contains at least one inductive heating element and at least one detection zone; (b) providing on said device droplets comprising a target nucleic acid, wherein said droplets comprise a subset of droplets that contains an agent for target nucleic acid detection and a subset of droplets that does not contain said agent for target nucleic acid detection; (c) amplifying the target nucleic acid in each said droplet in parallel; (d) quantitating the amplified target nucleic acid in said subset of droplets containing said agent through detection of said agent; and (e) after a desired amount of said target nucleic acid has been obtained in said subset of droplets containing an agent, recovering at least one droplet from said subset of droplets not containing an agent for further analyzing or processing. Yet another aspect of the invention generally pertains to an automated amplification method which comprises (a) providing an electrowetting-based device for effecting electrowetting-mediated droplet manipulations, and further wherein said device comprises at least one inductive heating element and at least one detection zone; (b) providing on said device at least 2 droplets which each comprise a different target nucleic acid, and further wherein said droplets comprise an agent for target nucleic acid detection comprising a DNA binding dye; (c) thermocycling said droplets in parallel, wherein said thermocycling amplifies said target nucleic acid; (d) quantitating the amplified target nucleic acid in said droplets through detection of the DNA binding dye; and (e) after a desired amount of said target nucleic acid has been obtained in said droplets, recovering at least one droplet for further analyzing or processing.

Yet another embodiment of the invention generally pertains to an automated amplification method which comprises (a) providing an electrowetting-based device for effecting electrowetting-mediated droplet manipulations, and further wherein said device contains at least one inductive heating element and at least one detection zone; (b) providing on said device at least 2 droplets which each comprise a different target nucleic acid, and further wherein said droplets comprise an agent for target nucleic acid detection comprising a DNA binding dye; (c) amplifying the target nucleic acid in each said droplet in parallel; (d) quantitating the amplified target nucleic acid in said subset of droplets containing said agent through detection of the DNA binding dye; and (e) after a desired amount of said target nucleic acid has been obtained in said droplets, recovering at least one droplet for further analyzing or processing. Yet another aspect of the invention generally pertains to an automated PCR amplification method which comprises (a) providing an electrowetting-based device for effecting electrowetting-mediated droplet manipulations, and further wherein said device contains at least one inductive heating element and at least one detection zone; (b) providing on said device at least 2 droplets which each comprise a different target nucleic acid, and further wherein said droplets comprise an agent for target nucleic acid detection; (c) amplifying the target nucleic acid in each said droplet in parallel; (d) quantitating the amplified target nucleic acid in said subset of droplets containing said agent through detection of said agent; and (e) after a desired amount of said target nucleic acid has been obtained in said droplets, recovering at least one droplet for further analyzing or processing.

In some embodiments, said device may comprise a planar array of electrodes. In some embodiments, said electrodes may be squares, optionally 5 mm by 5 mm.

### BRIEF DESCRIPTION OF THE SEVERAL VIEWS OF THE DRAWING

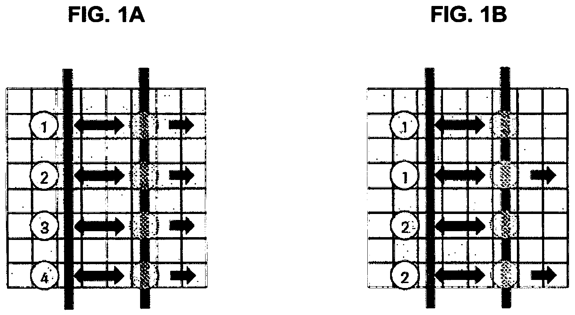

**FIG. 1A-1B** provides two example implementations of qPCR amplification on an electrowetting platform. **FIG. 1A** provides a representation of a qPCR amplification reaction in which there is only one droplet reaction for each of four different starting samples, labeled 1-4 in **FIG. 1A. FIG. 1B** provides a representation of a qPCR amplification reaction in which there are two droplet reactions for each of two different starting samples, labeled 1 and 2 in **FIG. 1B**.

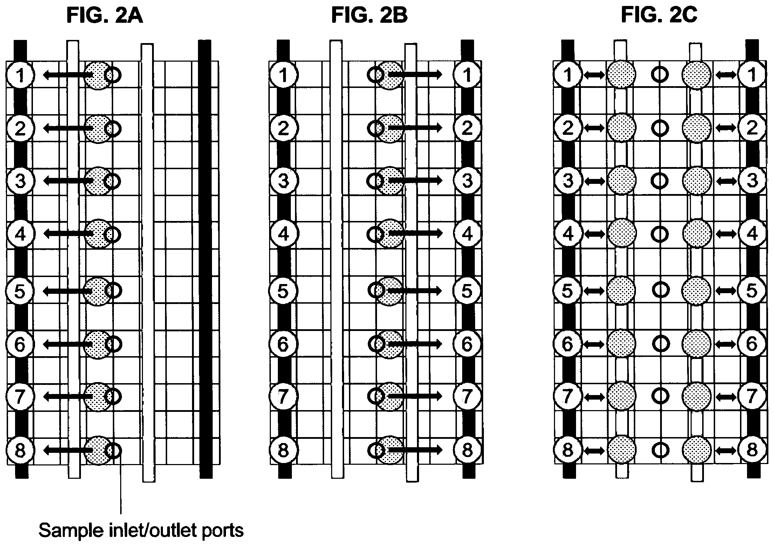

**FIG. 2A-2C** provides one example implementation of qPCR amplification on an electrowetting platform. **FIG. 2A-2C** provides a representation of a qPCR amplification eight different starting samples, labeled 1-8 in **FIG**. **2A-2C****.**

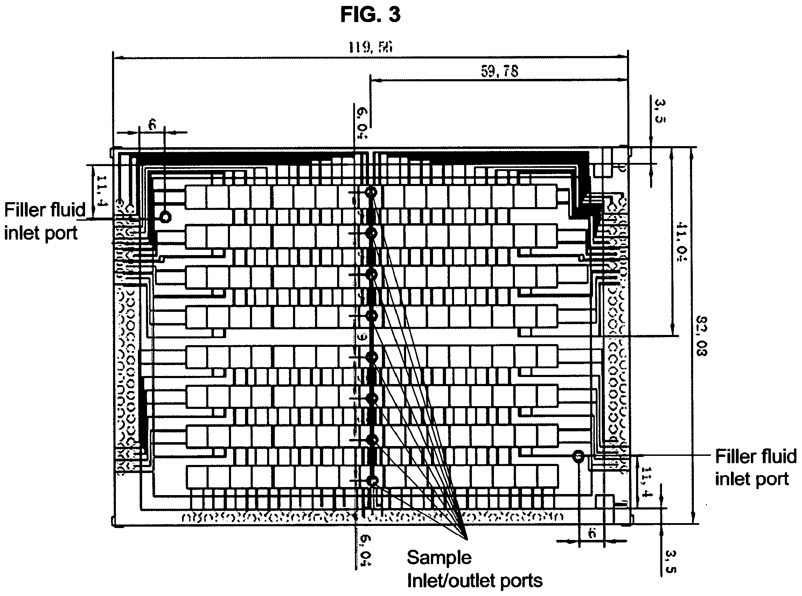

**FIG. 3** provides one design example of an electrowetting device which may be used with the methods, processes, and systems described herein. In **FIG. 3**, the dimensions are indicated in mm, and the "," is a decimal point.

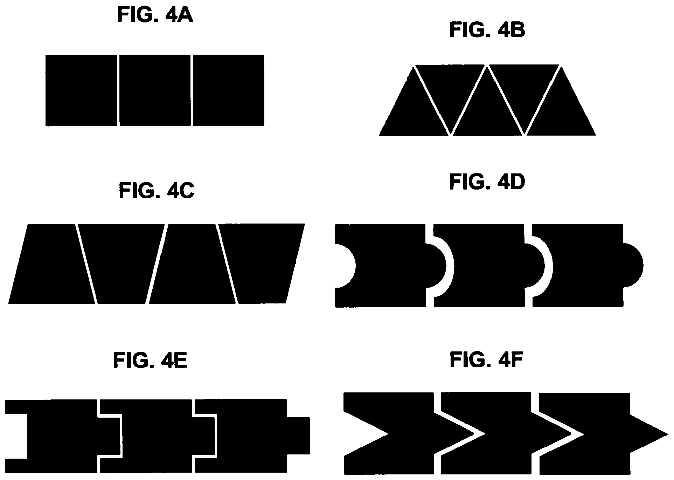

**FIG. 4A-4F** provides nonlimiting examples of electrode shapes that may be used with an electrowetting-based device as described herein. **FIG. 4A** presents an example of square shaped electrodes. **FIG. 4B** presents an example of triangular shaped electrodes. **FIG. 4C** presents an example of trapezoidal shaped electrodes. **FIG. 4D, FIG. 4E**, and **FIG. 4F** present examples of irregular shaped electrodes. Furthermore, **FIG. 4D, FIG. 4E,** and **FIG. 4F** present examples wherein adjacent electrodes may comprise portions of said electrodes that may be interdigitated.

### DETAILED DESCRIPTION OF THE INVENTION

### DEFINITIONS

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

The terms "droplet" and "microdroplet" are understood to refer to the same entity and to mean a defined volume of liquid. The droplet may be bounded by filler fluid, e.g. an oil, or by a gas such as air, but it is understood that the droplet is a defined volume of a liquid. The droplet also may take the form of various shapes and sizes, but it is understood that the droplet is a defined volume of liquid. Droplets may contain but are not limited to containing nucleic acids, target nucleic acids, detection agents, reaction mixtures, probes, samples, chemicals, proteins, cells, aqueous reagents, non-aqueous reagents, biological samples, and/or biological samples that contain biomarkers of interest.

The term "subset of droplets" refers to a grouping of any amount of droplets that may be defined by the presence or absence of a characteristic component. For example, a subset of droplets may comprise 1 or more, 2 or more, 10 or more, 100 or more, 1000 or more, or 10,000 or more droplets. A subset of droplets may all be defined by the presence of a detection agent, or a lack thereof, for example.

The term "filler fluid" refers to a fluid that is sufficiently immiscible with a droplet so as to render the droplet subject to electrowetting-mediated droplet manipulations. The filler fluid may be, for example, a low viscosity oil, such as a silicone-based oil or a fluorocarbon-based oil.

The terms "nucleic acid" and "nucleic acid molecule" may be used interchangeably throughout the disclosure. The term generally refers to polymers of nucleotides (e.g., ribonucleotides, deoxyribonucleotides, nucleotide analogs etc.) and comprising deoxyribonucleic acids (DNA), ribonucleic acids (RNA), DNA-RNA hybrids, oligonucleotides, polynucleotides, aptamers, peptide nucleic acids (PNAs), PNA-DNA conjugates, PNA-RNA conjugates, etc., that comprise nucleotides covalently linked together, either in a linear or branched fashion. A nucleic acid is typically single-stranded or double-stranded and will generally contain phosphodiester bonds, although in some cases, nucleic acid analogs are included that may have alternate backbones, including, for example, phosphoramide (Beaucage et al. (1993) Tetrahedron 49(10): 1925); phosphorothioate (Mag et al. (1991) Nucleic Acids Res. 19:1437; and U.S. Pat. No. 5,644,048), phosphorodithioate (Briu et al. (1989) J. Am. Chem. Soc. 111:2321), O-methylphophoroamidite linkages (see Eckstein, Oligonucleotides and Analogues: A Practical Approach, Oxford University Press (1992)), and peptide nucleic acid backbones and linkages (see, Egholm (1992) J. Am. Chem. Soc. 114:1895). Other analog nucleic acids include those with positively charged backbones (Denpcy et al. (1995) Proc. Natl. Acad. Sci. USA 92: 6097); non-ionic backbones (U.S. Pat. Nos. 5,386,023, 5,637,684, 5,602,240, 5,216,141 and 4,469,863) and non-ribose backbones, including those described in U.S. Pat. Nos. 5,235,033 and 5,034,506. Nucleic acids containing one or more carbocyclic sugars are also included within the definition of nucleic acids (see Jenkins et al. (1995) Chem. Soc. Rev. pp. 169-176), and analogs are also described in, e.g., Rawls, C 8c E News Jun. 2, 1997 page 35. These modifications of the ribose-phosphate backbone may be done to facilitate the addition of additional moieties such as labels, or to alter the stability and half-life of such molecules in physiological environments.

In addition to the naturally occurring heterocyclic bases that are typically found in nucleic acids (e.g., adenine, guanine, thymine, cytosine, and uracil), nucleotide analogs also may include non-naturally occurring heterocyclic bases, such as those described in, e.g., Seela et al. (1999) Helv. Chim. Acta 82:1640. Certain bases used in nucleotide analogs act as melting temperature (Tm) modifiers. For example, some of these include 7-deazapurines (e.g., 7-deazaguanine, 7-deazaadenine, etc.), pyrazolo[3,4-d]pyrimidines, propynyl-dN (e.g., propynyl-dU, propynyl-dC, etc.), and the like, see, e.g., U.S. Pat. No. 5,990,303. Other representative heterocyclic bases include, e.g., hypoxanthine, inosine, xanthine; 8-aza derivatives of 2-aminopurine, 2,6-diaminopurine, 2-amino-6-chloropurine, hypoxanthine, inosine and xanthine; 7-deaza-8-aza derivatives of adenine, guanine, 2-aminopurine, 2,6-diaminopurine, 2-amino-6-chloropurine, hypoxanthine, inosine and xanthine; 6-azacytidine; 5-fluorocytidine; 5-chlorocytidine; 5-iodocytidine; 5-bromocytidine; 5-methylcytidine; 5-propynylcytidine; 5-bromovinyluracil; 5-fluorouracil; 5-chlorouracil; 5-iodouracil; 5-bromouracil; 5-trifluoromethyluracil; 5-methoxymethyluracil; 5-ethynyluracil; 5-propynyluracil, and the like.

The terms nucleic acid and nucleic acid molecule also may generally refer to oligonucleotides, oligos, polynucleotides, genomic DNA, mitochondrial DNA (mtDNA), complementary DNA (cDNA), bacterial DNA, viral DNA, viral RNA, RNA, message RNA (mRNA), transfer RNA (tRNA), ribosomal RNA (rRNA), siRNA, catalytic RNA, clones, plasmids, M13, PI, cosmid, bacteria artificial chromosome (BAC), yeast artificial chromosome (YAC), amplified nucleic acid, amplicon, PCR product and other types of amplified nucleic acid, RNA/DNA hybrids and PNAs, all of which can be in either single- or double-stranded form, and unless otherwise limited, would encompass known analogs of natural nucleotides that can function in a similar manner as naturally occurring nucleotides and combinations and/or mixtures thereof. Thus, the term "nucleotides" refers to both naturally-occurring and modified/nonnaturally-occurring nucleotides, including nucleoside tri, di, and monophosphates as well as monophosphate monomers present within polynucleic acid or oligonucleotide. A nucleotide may also be a ribo; 2'-deoxy; 2',3'-deoxy as well as a vast array of other nucleotide mimics that are well-known in the art. Mimics include chain-terminating nucleotides, such as 3'-0-methyl, halogenated base or sugar substitutions; alternative sugar structures including nonsugar, alkyl ring structures; alternative bases including inosine; deaza-modified; chi, and psi, linker-modified; mass label-modified; phosphodiester modifications or replacements including phosphorothioate, methylphosphonate, boranophosphate, amide, ester, ether; and a basic or complete internucleotide replacements, including cleavage linkages such a photocleavable nitrophenyl moieties.

A "nucleoside" refers to a nucleic acid component that comprises a base or basic group (comprising at least one homocyclic ring, at least one heterocyclic ring, at least one aryl group, and/or the like) covalently linked to a sugar moiety (a ribose sugar or a deoxyribose sugar), a derivative of a sugar moiety, or a functional equivalent of a sugar moiety (e.g. a carbocyclic ring). For example, when a nucleoside includes a sugar moiety, the base is typically linked to a l'-position of that sugar moiety. As described above, a base can be a naturally occurring base or a non-naturally occurring base. Exemplary nucleosides include ribonucleosides, deoxyribonucleosides, dideoxyribonucleosides and carbocyclic nucleosides.

A "purine nucleotide" refers to a nucleotide that comprises a purine base, whereas a "pyrimidine nucleotide" refers to a nucleotide that comprises a pyrimidine base.

A "modified nucleotide" refers to rare or minor nucleic acid bases, nucleotides and modifications, derivations, or analogs of conventional bases or nucleotides and includes synthetic nucleotides having modified base moieties and/or modified sugar moieties (see, Protocols for Oligonucleotide Conjugates, Methods in Molecular Biology, Vol. 26 (Suhier Agrawal, Ed., Humana Press, Totowa, N.J., (1994)); and Oligonucleotides and Analogues, A Practical Approach (Fritz Eckstein, Ed., IRL Press, Oxford University Press, Oxford).

"Oligonucleotide" as used herein refers to linear oligomers of natural or modified nucleosidic monomers linked by phosphodiester bonds or analogs thereof. Oligonucleotides include deoxyribonucleosides, ribonucleosides, anomeric forms thereof, PNAs, and the like, capable of specifically binding to a target nucleic acid. Usually monomers are linked by phosphodiester bonds or analogs thereof to form oligonucleotides ranging in size from a few monomeric units, e.g., 3-4, to several tens of monomeric units, e.g., 40-60. Whenever an oligonucleotide is represented by a sequence of letters, such as "ATGCCTG," it will be understood that the nucleotides are in 5'-3' order from left to right and that "A" denotes deoxyadenosine, "C" denotes deoxycytidine, "G" denotes deoxyguanosine, "T" denotes deoxythymidine, and "U" denotes the ribonucleoside, uridine, unless otherwise noted. Usually oligonucleotides comprise the four natural deoxynucleotides; however, they may also comprise ribonucleosides or non-natural nucleotide analogs. Where an enzyme has specific oligonucleotide or polynucleotide substrate requirements for activity, e.g., single stranded DNA, RNA/DNA duplex, or the like, then selection of appropriate composition for the oligonucleotide or polynucleotide substrates is well within the knowledge of one of ordinary skill.

As used herein "oligonucleotide primer", or simply "primer", refers to a polynucleotide sequence that hybridizes to a sequence on a target nucleic acid template and may facilitate the detection or amplification of a target nucleic acid. In amplification processes, an oligonucleotide primer serves as a point of initiation of nucleic acid synthesis. In non-amplification processes, an oligonucleotide primer may be used to create a structure that is capable of being cleaved by a cleavage agent. Primers can be of a variety of lengths and are often less than 50 nucleotides in length, for example 12-25 nucleotides, in length. The length and sequences of primers for use in PCR can be designed based on principles known to those of skill in the art.

The term "oligonucleotide probe" as used herein refers to a polynucleotide sequence capable of hybridizing or annealing to a target nucleic acid of interest and allows for the specific detection of the target nucleic acid.

Nucleic acids are "extended" or "elongated" when additional nucleotides are incorporated into the nucleic acids, for example by a nucleotide incorporating biocatalyst, at the 3' end of a nucleic acid.

As used herein, the terms "hybridization" and "annealing" and the like are used interchangeably and refer to the base-pairing interaction of one polynucleotide with another polynucleotide (typically an antiparallel polynucleotide) that results in formation of a duplex or other higher-ordered structure, typically termed a hybridization complex. The primary interaction between the antiparallel polynucleotide molecules is typically base specific, e.g., A/T and G/C, by Watson/Crick and/or Hoogsteen-type hydrogen bonding. It is not a requirement that two polynucleotides have 100% complementarity over their full length to achieve hybridization. In some aspects, a hybridization complex can form from intermolecular interactions, or alternatively, can form from intramolecular interactions.

The term "complementary" means that one nucleic acid is identical to, or hybridizes selectively to, another nucleic acid molecule. Selectivity of hybridization exists when hybridization occurs that is more selective than total lack of specificity. Typically, selective hybridization will occur when there is at least about 55% identity over a stretch of at least 14-25 nucleotides, preferably at least 65%, more preferably at least 75%, and most preferably at least 90%. Preferably, one nucleic acid hybridizes specifically to the other nucleic acid. See M. Kanehisa, Nucleic Acids Res. 12:203 (1984).

A primer that is "perfectly complementary" has a sequence fully complementary across the entire length of the primer and has no mismatches. The primer is typically perfectly complementary to a portion (subsequence) of a target sequence and/or target nucleic acid. A "mismatch" refers to a site at which the nucleotide in the primer and the nucleotide in the target nucleic acid with which it is aligned are not complementary. The term "substantially complementary" when used in reference to a primer means that a primer is not perfectly complementary to its target sequence; instead, the primer is only sufficiently complementary to hybridize selectively to its respective strand at the desired primer-binding site.

The term "target nucleic acid" as used herein is intended to mean any nucleic acid whose presence is to be detected, measured, amplified, and/or subject to further assays and analyses. The term generally refers to a nucleic acid to which a primer nucleic acid can hybridize and be extended under suitable conditions. In the context of nucleic acid amplification, the "target nucleic acid" is preferably a region of double stranded nucleic acid, consisting of the sequences at least partially complementary to at least two primer sequences and the intervening sequence. A target can also be a single stranded nucleic acid, consisting of a sequence at least partially complementary to one primer and a sequence partially identical to the second primer. Target nucleic acids can exist as isolated nucleic acid fragments or be a part of a larger nucleic acid fragment. Target nucleic acids can be derived or isolated from essentially any source, such as cultured microorganisms, uncultured microorganisms, complex biological mixtures, biological samples, tissues, sera, ancient or preserved tissues or samples, environmental isolates or the like. Further, target nucleic acids optionally include or are derived from cDNA, RNA, genomic DNA, cloned genomic DNA, genomic DNA libraries, enzymatically fragmented DNA or RNA, chemically fragmented DNA or RNA, physically fragmented DNA or RNA, or the like.

The presence or absence of a target nucleic acid can be measured quantitatively or qualitatively. Target nucleic acids can come in a variety of different forms including, for example, simple or complex mixtures, or in substantially purified forms. For example, a target nucleic acid can be part of a sample that contains other components or can be the sole or major component of the sample. Also a target nucleic acid can have either a known or unknown sequence. The target nucleic acid may or may not be present in a droplet before amplification begins.

The term "amplification reaction" refers to any *in vitro* means for amplifying the copies of a target sequence of nucleic acid.

The terms "amplification" and "amplifying" the like refer generally to any process that results in an increase in the copy number of a molecule or set of related molecules. Components of an amplification reaction may include, but are not limited to, e.g., primers, a polynucleotide template, nucleic acid polymerase, nucleotides, dNTPs and the like. The term "amplifying" typically refers to an "exponential" increase in target nucleic acid. However, "amplifying" as used herein can also refer to linear increases in the numbers of a select target sequence of nucleic acid. Amplification typically starts from a small amount of a target nucleic acid (e.g. a single copy of a target nucleic acid), where the amplified material is typically detectable. Amplification of target nucleic acid encompasses a variety of chemical and enzymatic processes. The generation of multiple DNA copies from one or a few copies of a target nucleic acid may be effected by a polymerase chain reaction (PCR), a hot start PCR, a strand displacement amplification (SDA) reaction, a transcription mediated amplification (TMA) reaction, a nucleic acid sequence-based amplification (NASBA) reaction, or a ligase chain reaction (LCR). Amplification is not limited to the strict duplication of the starting target nucleic acid. For example, the generation of multiple cDNA molecules from a limited amount of viral RNA in a sample using RT-PCR is a form of amplification. Furthermore, the generation of multiple RNA molecules from a single DNA molecule during the process of transcription is also a form of amplification. Amplification may optionally followed by additional steps, for example, but not limited to, labeling, sequencing, purification, isolation, hybridization, size resolution, expression, detecting and/or cloning.

The term "amplification cycle" refers to the steps of an amplification reaction that are necessary to increase the amount of target nucleic acid. For example, using a standard PCR thermocycling protocol, a single amplification cycle would include the melting, annealing, and extension steps. In this example, if the concentration of the target nucleic acid were to be measured at the end of this amplification cycle, it would be measured after the extension step had been completed.

"Polymerase chain reaction" or "PCR" refers to a method whereby a specific segment or subsequence of a target double-stranded DNA, is amplified in a geometric progression. PCR is well known to those of skill in the art; see, e.g., U.S. Patent Nos. 4,683,195 and 4,683,202; and PCR Protocols: A Guide to Methods and Applications, Innis et al, eds, 1990.

The terms "real-time PCR" or "kinetic PCR" refer to real-time detection and/or quantitation of amplicon generated in a PCR.

The term "hot start amplification" refers to a modified form of PCR which can avoid non-specific amplification of DNA by inactivating the nucleic acid polymerase, e.g., Taq polymerase, at lower temperatures. Nucleic acid polymerase activity can be inhibited at these temperatures through different mechanisms, including antibody interaction, chemical modification, and aptamer technology. At permissive reaction temperatures reached during PCR cycling, the nucleic acid polymerase dissociates from its inhibitor and commences polymerization. Also, hot start PCR can be accomplished by omitting the nucleic acid polymerase from the reaction, placing all tubes onto the PCR machine, and then adding nucleic acid polymerase only when the reaction has reached 95°C. Additionally, other hot start PCR methods attempt to keep one or more components of the PCR separate from each other. This can be accomplished by freezing a PCR mixture containing primers, template, nucleotides, and water. Once frozen, the remaining PCR components, nucleic acid polymerase, buffer and MgCl₂ are pipetted on top and the tube transferred to a PCR machine already at the denaturation temperature. As the frozen layer melts the components are mixed by thermal currents. Additionally, wax beads can be used to form a layer separating two halves of a PCR mixture. This can be accomplished by pipetting a primer, template, nucleotide and water mixture into a PCR tube, adding a wax bead, melting the wax bead and allowing the mixture to cool forming an impenetrable wax barrier above the PCR mixture. Subsequent pipetting of the remaining PCR components over the wax layer complete the PCR whilst keeping components apart. Only when the wax melts above the primer annealing temperature do the two halves mix and allow the PCR to occur. Hot Start PCR can significantly reduce nonspecific priming, the formation of primer dimers, and often, increases product yields.

An "isothermal amplification reaction" refers to an amplification reaction wherein the temperature does not significantly change during the amplification reaction.

Depending on the method of isothermal amplification of nucleic acids, different enzymes are required for the amplification reaction. Known isothermal methods for amplification of nucleic acids are e.g. helicase-dependent amplification (HDA) (Vincent et al.; "Helicase-dependent isothermal DNA amplification", EMBO Reports 5(8): 795-800 (2004)), thermostable HDA (tHDA) (An, et al., "Characterization of a Thermostable UvrD Helicase and Its Participation in Helicase-dependent Amplification", J. Biol. Chem. 280(32): 28952-28958(2005)), strand displacement amplification (SDA) (Walker, et al., "Strand displacement amplification--an isothermal, in vitro DNA amplification technique," Nucleic Acids Res. 20(7):1691-6 (1992)), multiple displacement amplification (MDA) [Dean, et al., "Comprehensive human genome amplification using multiple displacement amplification", PNAS 99(8): 5261-5266 (2002)), rolling circle amplification (Liu, et al., "Rolling circle DNA synthesis: Small circular oligonucleotides as efficient templates for DNA polymerases," J. Am. Chem. Soc. 118:1587-1594 (1996)), single primer isothermal amplification (SPIA) [Dafforn, et al., "Linear mRNA amplification from as little as 5 ng total RNA for global gene expression analysis", Biotechniques 37(5):854-7 (2004)] restriction aided RCA [Wang, et al., "DNA amplification method tolerant to sample degradation", Genome Res. 14:2357-2366 (2004)], transcription mediated amplification (TMA) [Vuorinen, et al., "Direct detection of Mycobacterium tuberculosis complex in respiratory specimens by Gen-Probe Amplified Mycobacterium Tuberculosis Direct Test and Roche Amplicor PCR Mycobacterium Tuberculosis Test. J. Clin. Microbiol. 33: 1856-1859 (1995)], Nucleic Acid Sequence Based Amplification (NASBA) [Kievits, et al., "NASBA, isothermal enzymatic in vitro nucleic acid amplification optimized for the diagnosis of HIV-1 infection. J. Virol. Methods 35:273-286 (1991)] and amplification reactions using nicking enzymes, nicking enzyme amplification reaction (NEAR) [Maples, et al., "Nicking and extension amplification reaction for the exponential amplification of nucleic acids", US2009017453], amplification reactions using recombination proteins, recombinase polymerase amplification (RPA) [Piepenburg, et al., "DNA Detection Using Recombination Proteins", PLoSBiol. 4(7): e204 (2004)], and Loop-mediated isothermal amplification (LAMP) [Notomi, et al., "Loop-mediated isothethial amplification of DNA", NAR 28(12): e63 (2000)] wherein the at least one mesophilic enzyme for amplifying nucleic acids under isothermal conditions is selected from the group consisting of helicase, mesophilic polymerases, mesophilic polymerases having strand displacement activity, nicking enzymes, recombination proteins, ligases, glycosylases and nucleases.

"Helicases" are known by those skilled in the art. They are proteins that move directionally along a nucleic acid phosphodiester backbone, separating two annealed nucleic acid strands (e.g. DNA, RNA, or RNA-DNA hybrid) using energy derived from hydrolysis of NTPs or dNTPs, preferably ATP or dATP. Based on the presence of defined helicase motifs, it is possible to attribute a helicase activity to a given protein. A helicase may be selected from the group comprising but not limited to helicases from different families (superfamily I helicases (e.g. dda, perA, F-plasmid tral protein helicase, uvrD, superfamily II helicases (e.g. recQ, NS3-helicase), superfamily III helicases (e.g. AAV rep Helicase), helicases from dnaB-like superfamily (e.g. T7 phage helicase) or helicases form rho-like superfamily

A "mesophilic polymerase" refers to a polymerase that may be used to effect isothermal amplification. A mesophilic polymerase may be selected from the group comprising but not limited to phi29-polymerase, Bst-polymerase, Gst-polymerase, PyroPhage polymerase, Klenow polymerase, DisplaceAce Polymerase. All enzymes may be modified e.g. by eliminating nuclease activities or chemical modifications.

The term "real time quantitative PCR" ("qPCR") refers to methods that can be utilized to determine the quantity of a target nucleic acid present in a sample by measuring the amount of amplification product formed during or after any step of the amplification process itself. Quantitation may proceed through use of various detection agents, many of which are described herein.

A "nucleic acid polymerase" refers to an enzyme that catalyzes the incorporation of nucleotides into a nucleic acid. Exemplary nucleic acid polymerases include DNA polymerases, RNA polymerases, chimeric DNA polymerases, terminal transferases, reverse transcriptases, telomerases and the like. A polymerase for use with the device and method described herein includes KAPA HiFi DNA polymerase and RMS Z05 DNA polymerase from *Thermus* species Z05. A DNA polymerase can be a modified naturally occurring Type A polymerase selected from any species of the genus *Meiothermus,* any species of the genus *Thermotoga,* or any species of the genus *Thermomicrobium.* In some embodiments, a naturally-occurring polymerase suitable for the present invention is isolated from *Bacillus stearothermophilus, Sphaerobacter thermophilus, Dictoglomus thermophilum,* or *Escherichia coli.* In some embodiments, a naturally-occurring polymerase suitable for the present invention is isolated from *Thermus aquaticus, Thermus thermophilus, Thermus caldophilus,* or *Thermus filiformis.* The modification may include one or more amino acid changes selected for certain improved properties as described herein (US9315787). In some embodiments, the polymerase is *Thermus aquaticus (Taq)* polymerase. In some embodiments, the polymerase is the mutant Taq-E507K polymerase.

Chimeric DNA polymerases in accordance with the present invention may be engineered from any DNA polymerases, in particular, thermostable polymerases. Typically, DNA polymerases are grouped into six families: A, B, C, D, X and Y. Families A, B, C are grouped based on their amino acid sequence homologies to *E. coli* polymerases I, II, and III, respectively. Family X has no homologous *E. coli* polymerases. In some embodiments, DNA polymerases suitable for the present invention are family B DNA polymerases. Family B polymerases include, but are not limited to, *E. coli* pol II, archaeal polymerases, PRD1, phi29, M2, T4 bacteriophage DNA polymerases, eukaryotic polymerases α, Δ, ε, and many viral polymerases. In some embodiments, DNA polymerases suitable for the invention are archaeal polymerases (e.g., euryarchaeal polymerases).

Suitable exemplary archaeal polymerases include, but are not limited to, DNA polymerases from archaea (e.g., *Thermococcus litoralis* (Vent^{™}, GenBank: AAA72101), *Pyrococcus furiosus* (Pfu, GenBank: D12983, BAA02362), *Pyrococcus woesii, Pyrococcus* GB-D (Deep Vent^{™}, GenBank: AAA67131), *Thermococcus kodakaraensis* KODI (KOD, GenBank: BD175553, BAA06142; *Thermococcus* sp. strain KOD (Pfx, GenBank: AAE68738)), *Thermococcus gorgonarius* (Tgo, Pdb: 4699806), *Sulfolobus solataricus* (GenBank: NC002754, P26811), *Aeropyrum pernix* (GenBank: BAA81109), *Archaeglobus fulgidus* (GenBank: 029753), *Pyrobaculum aerophilum* (GenBank: AAL63952), *Pyrodictium occultum* (GenBank: BAA07579, BAA07580), *Thermococcus* 9 degree Nm (GenBank: AAA88769, Q56366), *Thermococcus fumicolans* (GenBank: CAA93738, P74918), *Thermococcus hydrothermalis* (GenBank: CAC18555), *Thermococcus* sp. GE8 (GenBank: CAC12850), *Thermococcus* sp. JDF-3 (GenBank: AX135456; WO0132887), *Thermococcus* sp. TY (GenBank: CAA73475), *Pyrococcus abyssi* (GenBank: P77916), *Pyrococcus glycovorans* (GenBank: CAC12849), *Pyrococcus horikoshii* (GenBank: NP 143776), *Pyrococcus* sp. GE23 (GenBank: CAA90887), *Pyrococcus* sp. ST700 (GenBank: CAC 12847), *Thermococcus pacificus* (GenBank: AX411312.1), *Thermococcus zilligii* (GenBank: DQ3366890), *Thermococcus aggregans, Thermococcus barossii, Thermococcus celer* (GenBank: DD259850.1), *Thermococcus profundus* (GenBank: E14137), *Thermococcus siculi* (GenBank: DD259857.1), *Thermococcus thioreducens, Thermococcus onnurineus* NA1, *Sulfolobus acidocaldarium, Sulfolobus tokodaii, Pyrobaculum calidifontis, Pyrobaculum islandicum* (GenBank: AAF27815), *Methanococcus jannaschii* (GenBank: Q58295), *Desulforococcus* species TOK, *Desulfurococcus, Pyrolobus, Pyrodictium, Staphylothermus, Vulcanisaetta, Methanococcus* (GenBank: P52025) and other archaeal B polymerases, such as GenBank AAC62712, P956901, BAAA07579)). Additional representative temperature-stable family A and B polymerases include, e.g., polymerases extracted from the thermophilic bacteria *Thermus* species (*e.g,,flavus, ruber, thermophilus, lacteus, rubens, aquaticus), Bacillus stearothermophilus, Thermotoga maritima, Methanothermus fervidus.*

Exemplary DNA polymerases characterized with high processivity, elongation rate, thermostability, salt or PCR enhancer tolerance include, but are not limited to, KOD polymerase, TNA1 polymerase, *Thermococcus* sp. 9 degrees N-7, T4, T7, or phi29. Exemplary DNA polymerases characterized with high fidelity include, but are not limited to, polymerases isolated from *Pyrococcus furiosus, P. abyssi, T. gorgonarius, T. litoralis, T. zilligii, T.* sp. GT, or P. sp. GB-D.

As non-limiting examples, KOD, Pfu, *T. gorgonarius, T. zilligii, T. litoralis* and *Thermococcus* sp. 9N-7 polymerases are used to engineer chimeric DNA polymerases, see the Examples herein (US9023633).

The term "thermostable polymerase," refers to an enzyme that is stable at elevated temperatures, is heat resistant, and retains sufficient activity to effect subsequent polynucleotide extension reactions and does not become irreversibly denatured (inactivated) when subjected to the elevated temperatures for the time necessary to effect denaturation of double-stranded nucleic acids. Thermostable DNA polymerases from thermophilic bacteria include, *e.g.,* DNA polymerases from *Thermotoga maritima, Thermus aquaticus, Thermus thermophilus, Thermus flavus, Thermus filiformis, Thermus* species Sps17, *Thermus* species Z05, *Thermus caldophilus, Bacillus caldotenax, Thermotoga neopolitana,* and *Thermosipho africanus.* In some embodiments, the polymerase is the DNA polymerase from *Thermus* species Z05 or *Thermus thermophilus.*

A "modified polymerase" refers to a polymerase in which at least one monomer differs from the reference sequence, such as a native or wild-type form of the polymerase or another modified form of the polymerase. Modifications include monomer insertions, deletions, and substitutions. Modified polymerases also include chimeric polymerases that have identifiable component sequences (e.g., structural or functional domains, etc.) derived from two or more parents. Also included within the definition of modified polymerases are those comprising chemical modifications of the reference sequence. The examples of modified polymerases include G46E E678G CS5 DNA polymerase, G46E L329A E678G CS5 DNA polymerase, G46E L329A D640G S671F CS5 DNA polymerase, G46E L329A D640G S671F E678G CS5 DNA polymerase, a G46E E678G CS6 DNA polymerase, Z05 DNA polymerase, ΔZ05 polymerase, AZ05-Gold polymerase, AZ05R polymerase, E615G Taq DNA polymerase, E678G TMA-25 polymerase, E678G TMA-30 polymerase, and the like.

The terms "agent", "detection agent", and "agent for detecting a target nucleic acid" generally refer to any reagent, compound, method, assay, probe, or fluorophore, for example, that can be used for the detection or the presence, absence, and/or amount of a target nucleic acid. Examples of such agents are defined below, but not limited to the below defined agents and include other agents and methods known in the art, such as hydrolysis probes, intercalating agents, primer probes, hybridization probes, analogues of nucleic acids, and detection-based assays, for example.

The term "hydrolysis probe" refers to an agent for detecting a target nucleic acid whose mechanism of action generally involves 5' to 3' nuclease activity. The term "5' to 3' nuclease activity" or "5'-3' nuclease activity" refers to an activity of a nucleic acid polymerase, typically associated with the nucleic acid strand synthesis, whereby nucleotides are removed from the 5' end of nucleic acid strand, e.g., *E. coli* DNA polymerase I has this activity, whereas the Klenow fragment does not. Some enzymes that have 5' to 3' nuclease activity are 5' to 3' exonucleases. Examples of such 5' to 3' exonucleases include: Exonuclease from B. subtilis, Phosphodiesterase from spleen, Lambda exonuclease, Exonuclease II from yeast, Exonuclease V from yeast, and Exonuclease from Neurospora crassa.

Further, the detection of a target nucleic acid utilizing the 5' to 3' nuclease activity can be performed by a "TAQMAN^{®}" or "5 '-nuclease assay", as described in U.S. Patent Nos. 5,210,015; 5,487,972; and 5,804,375; and Holland et al., 1988, Proc. Natl. Acad. Sci. USA 88: 7276-7280. In the TaqMan^{®} assay, labeled detection probes that hybridize within the amplified region are present during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

Any method suitable for detecting degradation product can be used in a 5' nuclease assay. Often, the detection probe is labeled with two fluorescent dyes, one of which is capable of quenching the fluorescence of the other dye. The dyes are attached to the probe, typically with the reporter or detector dye attached to the 5' terminus and the quenching dye attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Patent Nos. 5,491,063 and 5,571,673 describe alternative methods for detecting the degradation of a probe which occurs concomitant with amplification. Examples of fluorescent dyes for use with hydrolysis probes include, but are not limited to, FAM^{™}, TET^{™}, HEX^{™}, VIC^{™} CY3^{™}, CY5^{™}, fluorescein, rhodamine, OREGON GREEN^{®}, eosin, TEXAS RED^{™}, cyanine, indocarbocyanine, oxacarbocyanine, thiacarbocyanine, merocyanine, hydroxycoumarin, aminocoumarin, methoxycoumarin, CASCADE BLUE^{™}, PACIFIC BLUE^{™}, PACIFIC ORANGE^{™}, LUCIFER YELLOW^{™}, R-phycoerthrin, peridinin chlorophyll protein, FLUORX^{™}, BODIPY-fluorescein, CY2^{™}, CY3B^{™}, CY3.5^{™}, CY5.5^{™}, CY7^{™}, TRITC^{™}, lissamine rhodamine B, or allophycocyanin.

Fluorescent dyes may include dyes that are negatively charged, such as dyes of the fluorescein family, or dyes that are neutral in charge, such as dyes of the rhodamine family, or dyes that are positively charged, such as dyes of the cyanine family. Dyes of the fluorescein family include, e.g., 6-carboxy-fiuorescein (FAM), 2', 4, 4', 5', 7, 7'-hexachlorofiuorescein (HEX), TET, JOE, NAN and ZOE. Dyes of the rhodamine family include, e.g., Texas Red, ROX, Rl lO, R6G, and TAMRA or the rhodamine derivative JA270 (see, US Patent No. 6,184,379). FAM, HEX, TET, JOE, NAN, ZOE, ROX, Rl lO, R6G, and TAMRA are commercially available from, e.g., Perkin-Elmer, Inc. (Wellesley, MA, USA), and Texas Red is commercially available from, e.g., Molecular Probes, Inc. (Eugene, OR). Dyes of the cyanine family include, e.g., Cy2, Cy3, Cy5, Cy 5.5 and Cy7, and are commercially available from, e.g., Amersham Biosciences Corp. (Piscataway, NJ, USA).

A 5' nuclease assay for the detection of a target nucleic acid can employ any polymerase that has a 5' to 3' exonuclease activity. Thus, in some instances, the polymerases with 5'-nuclease activity are thermostable and thermoactive nucleic acid polymerases. Such thermostable polymerases include, but are not limited to, native and recombinant forms of polymerases from a variety of species of the eubacterial genera *Thermus, Thermatoga,* and *Thermosipho,* as well as chimeric forms thereof. For example, *Thermus* species polymerases that can be used include *Thermus aquaticus* (Taq) DNA polymerase, *Thermus thermophilus* (Tth) DNA polymerase, *Thermus* species Z05 (Z05) DNA polymerase, *Thermus* species spsl7 (spsl7), and *Thermus* species Z05 (e.g., described in U.S. Patent Nos. 5,405,774; 5,352,600; 5,079,352; 4,889,818; 5,466,591; 5,618,711; 5,674,738, and 5,795,762). *Thermatoga* polymerases that can be include, for example, *Thermatoga maritima* DNA polymerase and *Thermatoga neapolitana* DNA polymerase, while an example of a *Thermosipho* polymerase that can be used is *Thermosipho* africanus DNA polymerase. The sequences of *Thermatoga maritima* and *Thermosipho africanus* DNA polymerases are published in International Patent Publication No. WO 92/06200. The sequence of *Thermatoga neapolitana* may be found in International Patent Publication No. WO 97/09451.

In the 5' nuclease assay, the amplification detection is typically concurrent with amplification (i.e., "real-time"). In some instances the amplification detection is quantitative, and the amplification detection is real-time. In some instances, the amplification detection is qualitative (e.g., end-point detection of the presence or absence of a target nucleic acid). In some instances, the amplification detection is subsequent to amplification. In some instances, the amplification detection is qualitative, and the amplification detection is subsequent to amplification.

A "label" refers to a moiety attached (covalently or non-covalently), to a molecule and capable of providing information about the molecule. Exemplary labels include fluorescent labels, colorimetric labels, chemiluminescent labels, bioluminescent labels, radioactive labels, mass-modifying groups, antibodies, antigens, biotin, haptens, and enzymes (including peroxidase, phosphatase, etc.).

The term "DNA binding dye" or "DNA binding agent" generally refers to a molecule whose fluorescent signal is enhanced upon binding to or interacting with DNA. DNA binding agent produces a detectable signal directly or indirectly. The signal is detectable directly, such as by fluorescence or absorbance, or indirectly via a substituted label moiety or binding ligand attached to the DNA binding agent. For indirect detection any moiety or ligand that is detectably affected by proximity to double-stranded DNA is suitable. DNA binding dyes encompass both "intercalating agents" and "non-intercalating agents".

As used herein, an "intercalating agent" is an agent or moiety capable of non-covalent insertion between stacked base pairs in the nucleic acid double helix. Intercalating agents, such as ethidium bromide, fluoresce more intensely when intercalated into double-stranded DNA than when bound to single-stranded DNA, RNA, or in solution. Other intercalating agents exhibit a change in the fluorescence spectra when bound to double-stranded DNA. For example, actinomycin D fluoresces red when bound to single-stranded nucleic acids, and green when bound to a double-stranded template. Whether the detectable signal increases, decreases, or is shifted, as is the case with actinomycin D, any intercalating agent that provides a detectable signal that is distinguishable when the agent is bound to double-stranded DNA or unbound is suitable for use with any of the methods or devices described herein. For example, the interaction between DNA and another photoreactive psoralen, 4-aminomethyle-4-5'8-trimethylpsoralen (AMT) has been described (see Johnson et al., 1981, Photochem, & Photobiol., 33:785-791). According to the reference, both the absorption at long wavelengths and fluorescence, decline upon intercalation of AMT into the DNA helix.

"Non-intercalating agents" are also suitable. For example, Hoechst 33258 (Searle & Embrey, 1990, Nuc. Acids Res. 18(13):3753-3762) exhibits altered fluorescence with increasing amount of target. Hoechst 33258 is a member of a class of DNA-binding compounds commonly referred to as "groove binders." This group includes drugs like distamycin, netropsin and others. These compounds recognize and bind the minor groove of duplex DNA.

Examples of DNA binding agents, inclusive of both intercalating agents and non-intercalating agents, include but are not limited to ethidium bromide, SYBR^{™} Green, PICOGREEN^{®}, SYBR^{™} Gold, SYTO^{®} 9, SYTO^{®} 13, SYTO^{®} 16, SYTOX^{®} blue, chromomycin A3, Os[(bpy)2DPPZ]2+, BEBO, BOXTO, EVAGREEN^{®}, propidium iodide, chromomycin, mithramycin, thiazole orange, CYTRAK ORANGE^{™}, LDS 751, 7-AAD, SYTOX^{®} green, SYTOX^{®} orange, TOTO-3, DRAG5, DRAG7, ResoLight, acridine orange, Hoechst 33258, TOTO-1, YOYO-1, YO-PRO-1, TO-PRO-3, and 4',6-diamidino-2-phenylindole ("DAPI").

The term "primer probe" generally refers to a class of target nucleic acid detection agents that combine a primer and detection agent in a single molecule. Fluorescence emitted from primer-probes is generally detected and measured during the denaturation or extension phase of qPCR, depending on the type of primer-probe used. Some examples of primer probes include, but are not limited to, Scorpion probes, AMPLIFLUOR^{®} probes, Sunrise probes, LUX^{™} probes, cyclicon probes, and ANGLER^{®} probes.

Scorpion probes are generally used according to the following methodology. This method is described, for example, by Thelwell N., et al. Nucleic Acids Research, 28:3752-3761, 2000, wherein Scorpion probing mechanism is as follows. Step 1: initial denaturation of target and Scorpion stem sequence. Step 2: annealing of Scorpion primer to target. Step 3: extension of Scorpion primer produces double-stranded DNA. Step 4: denaturation of double-stranded DNA produced in step 3. This gives a single-stranded target molecule with the Scorpion primer attached. Step 5: on cooling, the Scorpion probe sequence binds to its target in an intramolecular manner. This is favored over the intermolecular binding of the complementary target strand. A Scorpion consists of a specific probe sequence that is held in a hairpin loop configuration by complementary stem sequences on the 5' and 3' sides of the probe. The fluorophore attached to the 5'-end is quenched by a moiety (normally methyl red) joined to the 3'-end of the loop. The hairpin loop is linked to the 5'-end of a primer via a PCR stopping sequence (stopper). After extension of the primer during PCR amplification, the specific probe sequence is able to bind to its complement within the same strand of DNA. This hybridization event opens the hairpin loop so that fluorescence is no longer quenched and an increase in signal is observed. The PCR stopping sequence prevents read-through that could lead to opening of the hairpin loop in the absence of the specific target sequence. Such read-through would lead to the detection of non-specific PCR products, e.g. primer dimers or mispriming events.

Sunrise probes, known by their commercial name AMPLIFLUOR^{™} probes, are similar to Scorpion probes in their mechanism of action. When the primer-probe is not bound, the hairpin structure is intact and the reporter transfers energy to the quencher via FRET-quenching. DNA amplification occurs after binding of the primer-probe to the target sequence. In the next step of denaturation, reporter and quencher are separated and, as a result, the emitted fluorescence of the donor is measured.

LUX^{™} primer probes act through a mechanism whereby the hairpin structure confers the ability to decrease the fluorescence signal when the primer-probe is free and increases the signal exponentially when it binds to its target sequence. The maximum fluorescence emission is generated after the incorporation of LUX^{™} primer-probes into double-stranded DNA. Fluorescence is measured during the extension phase.

Cyclicon primer probes act through a mechanism whereby in the absence of the target sequence, reporter and quencher molecules are in close proximity and energy transfer occurs via FRET-quenching. The binding of Cyclicon probes to DNA opens up the cyclic structure and leads to extension of the 3'-end primer-probe by DNA polymerase without any interference from the quencher. The 3'-end of the modified oligo is not extendible since it does not bind to the target DNA and because its 3'-end is blocked by a reporter. The separation between donor and acceptor molecules results in emission of fluorescence, which is measured during the extension phase.

ANGLER^{®} primer-probes work through a mechanism whereby, in solution, the primer-probe does not emit fluorescence since there is no donor fluorescent moiety close enough for FRET. When the ANGLER primer-probe binds to its target DNA during the annealing step, DNA polymerase starts the extension of the 3'-end reverse primer. Subsequently, during the denaturation phase, the specific sequence of the probe binds to the complementary region of newly amplified DNA, producing a dsDNA fragment in which SYBR gold dye can be intercalated to generate fluorescence. Hence, the emitted fluorescence is measured during the denaturation step in each cycle.

The term "hybridization probe" generally refers to an a method of detection that involves an agent or agent that are able bind to and/or recognize a specific sequence and/or a specific structural feature of a target nucleic acid. Hybridization probes may take the form of, but are not limited to, FRET hybridization probes, Molecular Beacon probes, HYBEACON^{™} probes, an MGB probe such as MGB-Pleiades and MGB-Eclipse, a RESONSENSE^{®} probe, or a Yin-Yang probe.

FRET Hybridization Probe test format is especially useful for various homogenous hybridization assays (Matthews, J. A., and Kricka, L. J., Analytical Biochemistry 169 (1988) 1-25). It is characterized by a pair of two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.

When annealed to the target sequence, the hybridization probes must sit very close to each other, in a head to tail arrangement. Usually, the gap between the labeled 3' end of the first probe and the labeled 5' end or the second probe is as small as possible, i.e. 1-5 bases. This allows for a close vicinity of the FRET donor compound and the FRET acceptor compound, which is typically 10-100 Å.

As an alternative to monitoring the increase in fluorescence of the FRET acceptor component, it is also possible to monitor fluorescence decrease of the FRET donor component as a quantitative measurement of a hybridization event.

In particular, the FRET Hybridization Probe format may be used in qPCR in order to detect the amplified target DNA. Among all detection formats known in the art of qPCR, the FRET-Hybridization Probe format has been proven to be highly sensitive, exact, and reliable (WO 97/46707; WO 97/46712; WO 97/46714).

As an alternative to the usage of two FRET hybridization probes, it is also possible to use a fluorescent-labeled primer and only one labeled oligonucleotide probe (Bernard, P. S., et al., Analytical Biochemistry 255 (1998) 101-107). In this regard, it may be chosen arbitrarily, whether the primer is labeled with the FRET donor or the FRET acceptor compound.

Besides PCR and qPCR, FRET hybridization probes are used for a "melting curve analysis". In such an assay, the target nucleic acid is amplified first in a typical PCR reaction with suitable amplification primers. The hybridization probes may already be present during the amplification reaction or added subsequently. After completion of the PCR-reaction, the temperature of the sample is constitutively increased, and fluorescence is detected as long as the hybridization probe was bound to the target DNA. At melting temperature, the hybridization probes are released from their target, and the fluorescent signal is decreasing immediately down to the background level. This decrease is monitored with an appropriate fluorescence versus temperature-time plot such that a first derivative value can be determined, at which the maximum of fluorescence decrease is observed.

"Molecular Beacons" may also be used as a component of a detection agent. With molecular beacons, a change in conformation of the probe as it hybridizes to a complementary region of the amplified product results in the formation of a detectable signal. The probe itself includes two sections: one section at the 5' end and the other section at the 3' end. These sections flank the section of the probe that anneals to the probe binding site and are complementary to one another. One end section is typically attached to a reporter dye and the other end section is usually attached to a quencher dye.

In solution, the two end sections can hybridize with each other to form a hairpin loop. In this conformation, the reporter and quencher dye are in sufficiently close proximity that fluorescence from the reporter dye is effectively quenched by the quencher dye. A hybridized probe, in contrast, results in a linearized conformation in which the extent of quenching is decreased. Thus, by monitoring emission changes for the two dyes, it is possible to indirectly monitor the formation of amplification product. Probes of this type and methods of their use is described further, for example, by Piatek, A. S., et al., Nat. Biotechnol. 16:359-63 (1998); Tyagi, S. and Kramer, F. R., Nature Biotechnology 14:303-308 (1996); and Tyagi, S. et al., Nat. Biotechnol. 16:49-53 (1998).

HYBEACON^{™} probes, as described by French et al., consist of single-stranded-oligonucleotide sequences containing fluorophore moieties attached to internal nucleotides, and a 3'-end blocker (3'-phosphate or octanediol), which prevents their PCR extension. The amount of fluorescence emitted from hybridized HYBEACONs^{™} when they bind to their target is considerable, and the fluorescent signal is generally measured during the extension phase. This system allows for melting curve analysis to be carried out to address the specificity of the amplified product and the efficiency of the reaction.

Minor groove binding ("MGB") probes generally consist of a probe such as Pleiades (Navarro et al. 89) or Eclipse (Navarro et al. 90) attached through their 3' or 5' ends to an MGB ligand. An MGB ligands are generally small molecule tripeptides, including dihydrocyclopyrooloindole tripeptide ("DIP") or 1,2-dihydro-(3H)-pyrrolo [3.2-e] indole-7-carboxylate ("CDPI") that form a non-covalent union with the minor groove of double-stranded DNA. This type of ligand selectively binds to AT-rich sequences, favoring the inclusion of aromatic rings by van der Waals and electrostatic interactions. This interaction produces very minimal distortion in the phosphodiester backbone but greatly stabilizes DNA structure.

RESONSENSE probes have a CY5.5^{®} fluorescent-Fluor at the 5'-end as an acceptor fluorescent moiety and a phosphate group at the 3'-end to prevent DNA polymerization. The qPCR reaction generally also contains the binding dye SYBR^{®} gold as a fluorescence donor, which intercalates into the DNA duplex formed by the probe and its target. In solution, fluorescence is not emitted from the probe due to the absence of a fluorescent donor close enough to the acceptor. During the annealing phase, energy transfer by FRET is produced as a result of simultaneous binding of the probe to the target and intercalation of the DNA dye into the probe-target duplex. The fluorescence signal is proportional to the concentration of target DNA sequences.

Yin-Yang probes are double-stranded probes that are composed of two complementary oligonucleotides of different lengths. The 5'-end of the longer positive strand is labeled with a fluorophore reporter and blocked with a phosphate group at its 3'-end, whereas the 3'-end of the shorter negative strand contains a fluorophore quencher. In solution, the shorter negative oligonucleotide, which acts as a competitor, forms a stable DNA duplex with the longer probe. This interaction prevents the fluorescent emission due to the fact that the reporter and quencher remain in close proximity. During the annealing phase, the shorter strand is displaced by the target leading to the emission of fluorescence.

"Nucleic acid analogues" may also be used for detection of a target nucleic acid. Nucleic acid analogues are compounds that are analogous (structurally similar) to naturally occurring RNA and DNA. An analogue may have alterations in its phosphate backbone, pentose sugar (either ribose or deoxyribose) or nucleobases. Normally, the analogues incorporate all of the advantages of native DNA but are more stable in biological fluids and have increased affinity for complementary nucleic acid targets. Some examples of nucleic acid analogues that can be used to detect a target nucleic acid include but are not limited to PNAs, locked nucleic acids ("LNAs"), Zip nucleic acids ("ZNAs^{™}"), Plexo primers, or Tiny Molecular Beacon probes. Nucleic acid analogues are generally inserted into a primer-probe in order to effect target nucleic acid detection.

The term "detection-based assay" generally refers to an assay or format wherein the presence or absence of a target nucleic acid may be detected. Examples of detection-based assays include, but are not limited to, invader assays, NASBA assays, and capacitance detection of a droplet and/or the target nucleic acid contained within a droplet. "Invader assays" (Third Wave Technologies, (Madison, Wis.)) are used for SNP genotyping and utilize an oligonucleotide, designated the signal probe, that is complementary to the target nucleic acid (DNA or RNA) or polymorphism site. A second oligonucleotide, designated the Invader Oligo, contains the same 5' nucleotide sequence, but the 3' nucleotide sequence contains a nucleotide polymorphism. The Invader Oligo interferes with the binding of the signal probe to the target nucleic acid such that the 5' end of the signal probe forms a "flap" at the nucleotide containing the polymorphism. This complex is recognized by a structure specific endonuclease, called the Cleavase enzyme. Cleavase cleaves the 5' flap of the nucleotides. The released flap binds with a third probe bearing FRET labels, thereby forming another duplex structure recognized by the Cleavase enzyme. This time the Cleavase enzyme cleaves a fluorophore away from a quencher and produces a fluorescent signal. For SNP genotyping, the signal probe will be designed to hybridize with either the reference (wild type) allele or the variant (mutant) allele. Unlike PCR, there is a linear amplification of signal with no amplification of the nucleic acid. Further details sufficient to guide one of ordinary skill in the art are provided by, for example, Neri, B. P., et al., Advances in Nucleic Acid and Protein Analysis 3826:117-125, 2000.

Another example of a detection based assay may be a nucleic acid sequence based amplification ("NASBA") assay. NASBA is a detection method using RNA as the template. A primer complementary to the RNA contains the sequence for the T7 promoter site. This primer is allowed to bind with the template RNA and Reverse Transcriptase (RT) added to generate the complementary strand from 3' to 5'. RNase H is subsequently added to digest away the RNA, leaving single stranded cDNA behind. A second copy of the primer can then bind the single stranded cDNA and make double stranded cDNA. T7 RNA polymerase is added to generate many copies of the RNA from the T7 promoter site that was incorporated into the cDNA sequence by the first primer. All the enzymes mentioned are capable of functioning at 41° C. (See, e.g., Compton, J. Nucleic Acid Sequence-based Amplification, Nature 350: 91-91, 1991.).

A detection based assay may also take the form of capacitive detection of target nucleic acid within a droplet. For instance, there is a linear relationship between DNA concentration and the change in capacitance that is evoked by the passage of nucleic acids across a 1-kHz electric field. This relationship has been found to be species independent. (See, e.g., Sohn, et al. (2000) Proc. Natl. Acad. Sci. U.S.A. 97:10687-10690). Thus, in certain devices, nucleic acids within the flow channel (e.g., the substantially circular flow channel of FIG. 1 or the reaction chambers of FIG. 2) are subjected to such a field to determine concentration of amplified product. Alternatively, solution containing amplified product is withdrawn and then subjected to the electric field.

The terms "detection zone" and "detection region" refer to a location wherein signal from one or more detection agents may be detected. Said detection of an agent or multiple agents may occur simultaneously or sequentially. The detection zone can be designed to detect a number of different signal types including, but not limited to, signals from radioisotopes, fluorophores, chromophores, electron dense particles, magnetic particles, spin labels, molecules that emit chemiluminescence, electrochemically active molecules, enzymes, cofactors, enzymes linked to nucleic acid probes and enzyme substrates. Illustrative detection methodologies suitable for use with the present device include, but are not limited to, light scattering, multichannel fluorescence detection, UV and visible wavelength absorption, luminescence, differential reflectivity, and confocal laser scanning. Additional detection methods that can be used in certain application include scintillation proximity assay techniques, radiochemical detection, fluorescence polarization, fluorescence correlation spectroscopy (FCS), time-resolved energy transfer (TRET), fluorescence resonance energy transfer (FRET) and variations such as bioluminescence resonance energy transfer (BRET). Additional detection options include electrical resistance, resistivity, impedance, and voltage sensing.

The detection zone can be in communication with one or more microscopes, diodes, light stimulating devices (e.g., lasers), photomultiplier tubes, processors and combinations of the foregoing, which cooperate to detect a signal associated with a particular event and/or agent. Often the signal being detected is an optical signal that is detected in the detection section by an optical detector. The optical detector can include one or more photodiodes (e.g., avalanche photodiodes), a fiber-optic light guide leading, for example, to a photomultiplier tube, a microscope, and/or a video camera (e.g., a CCD camera).

Detection zones can be microfabricated within the device, or can be a separate element. If the detector exists as a separate element and the device includes a plurality of detection zones, detection can occur within a single detection zone at any given moment. Alternatively, scanning systems can be used. For instance, certain automated systems scan the light source relative to the electrowetting-based device; other systems scan the emitted light over a detector, or include a multichannel detector. The device can be attached to a translatable stage and scanned under a microscope objective. A signal so acquired is then routed to a processor for signal interpretation and processing. Arrays of photomultiplier tubes can also be utilized. Additionally, optical systems that have the capability of collecting signals from all the different detection sections simultaneously while determining the signal from each section can be utilized.

A detector can include a light source for stimulating a reporter that generates a detectable signal. The type of light source utilized depends in part on the nature of the reporter being activated. Suitable light sources include, but are not limited to, lasers, laser diodes and high intensity lamps. If a laser is utilized, the laser can be utilized to scan across a set of detection sections or a single detection section. Laser diodes can be microfabricated into the device itself. Alternatively, laser diodes can be fabricated into another device that is placed adjacent to the microfluidic device being utilized to conduct a thermal cycling reaction such that the laser light from the diode is directed into the detection section.

Detection can involve a number of non-optical approaches as well. For example, the detector can also include, for example, a temperature sensor, a conductivity sensor, a potentiometric sensor (e.g., pH electrode) and/or an amperometric sensor (e.g., to monitor oxidation and reduction reactions).

The term "heating element" generally refers to any means by which temperature change, particularly heating, may be achieved on a device. For example, said heating elements may comprise a heater on the chip or a heater separate from the electrowetting-based device. Further, heating can be achieved through using contact heaters that are a separate element from the electrowetting-based device itself, or through heating elements that are fabricated as a part of the electrowetting-based device. A heating element may also be an inductive heating element, or a conductive heating element, either of which may be a separate element from the electrowetting-based device or fabricated as a part of the electrowetting-based device.

The term "reaction mixture" refers to a solution containing reagents necessary to carry out a given reaction. An "amplification reaction mixture", which refers to a solution containing reagents necessary to carry out an amplification reaction, typically contains oligonucleotide primers and a DNA polymerase or ligase in a suitable buffer. A "PCR reaction mixture" typically contains oligonucleotide primers, a thermostable DNA polymerase dNTP's, and a divalent metal cation in a suitable buffer. A reaction mixture is referred to as complete if it contains all reagents necessary to enable the reaction, and incomplete if it contains only a subset of the necessary reagents. It will be understood by one of skill in the art that reaction components are routinely stored as separate solutions, each containing a subset of the total components, for reasons of convenience, storage, stability, or to allow for application-dependent adjustment of the component concentrations, and, that reaction components are combined prior to the reaction to create a complete reaction mixture.

A "polymorphic marker" or "polymorphic site" is the locus at which divergence occurs. Preferred markers have at least two alleles, each occurring at frequency of greater than 1%, and more preferably greater than 10% or 20% of a selected population. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms.

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than {fraction (1/100)} or {fraction (1/1000)} members of the populations). A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

As used herein, the term "biomarker" or "biomarker of interest" refers to a biological molecule found in blood, other body fluids, or tissues that is a sign of a normal or abnormal process, or of a condition or disease (such as cancer). A biomarker may be used to see how well the body responds to a treatment for a disease or condition. In the context of cancer, a biomarker refers to a biological substance that is indicative of the presence of cancer in the body. A biomarker may be a molecule secreted by a tumor or a specific response of the body to the presence of cancer. Genetic, epigenetic, proteomic, glycomic, and imaging biomarkers can be used for cancer diagnosis, prognosis, and epidemiology. Such biomarkers can be assayed in non-invasively collected biofluids like blood or serum. Several gene and protein based biomarkers have already been used in patient care including but, not limited to, AFP (Liver Cancer), BCR-ABL (Chronic Myeloid Leukemia), BRCA1 / BRCA2 (Breast/Ovarian Cancer), BRAF V600E (Melanoma/Colorectal Cancer), CA-125 (Ovarian Cancer), CA19.9 (Pancreatic Cancer), CEA (Colorectal Cancer), EGFR (Non-small-cell lung carcinoma), HER-2 (Breast Cancer), KIT(Gastrointestinal stromal tumor), PSA (Prostate Specific Antigen) (Prostate Cancer), S100 (Melanoma), and many others. Biomarkers may be useful as diagnostics (to identify early stage cancers) and/or prognostics (to forecast how aggressive a cancer is and/or predict how a subject will respond to a particular treatment and/or how likely a cancer is to recur). Biomarkers of interest include, but are not limited to, such oncology biomarkers as AKAP4, ALK, APC, AR, BRAF, BRCA1, BRCA2, CCND1, CCND2, CCND3, CD274, CDK4, CDK6, CFB, CFH, CFI, DKK1, DPYD, EDNRB, EGFR, ERBB2, EPSTI1, ESR1, FCRL5, FGFR1, FGFR2, FGFR3, FLT3, FN14, HER2, HER4, HERC5, IDH1, IDH2, IDOl, KIF5B, KIT, KRAS, LGR5, LIV1, LY6E, LYPD3, MACC1, MET, MRD, MSI, MSLN, MUC16, MYC, NaPi3b, NRAS, PDGFRA, PDCD1LG2, RAF1, RNF43, NTRK1, NTSR1, OX40, PIK3CA, RET, ROS1,Septin 9,TERT,TFRC,TROP2,TP53, TWEAK, and UGT1A1.

The terms "electrowetting-based device" and "electrowetting device" refer to a device for manipulating droplets using electrowetting-mediated droplet manipulations that are based on the property of electrowetting. For an example of such an electrowetting-based device, see **FIG. 3**. For additional examples of electrowetting-based devices and/or an electrowetting device, see U.S. Pat. No. 8,409,417, entitled "Electrowetting based digital microfluidics", issued on Apr. 2, 2013 to Wuand U.S. Patent No. 8,926,811, entitled "Digital microfluidics based apparatus for heat-exchanging chemical processes", issued on Jan. 6, 2015 to Wu.

In some embodiments, an electrowetting-based device may comprise a planar array of electrodes. In some embodiments, an electrowetting-based device may comprise a biplanar array of electrodes. In some embodiments, an electrowetting-based device may comprise square-shaped electrodes, e.g., wherein said electrodes may be about 5 mm by 5 mm. In some embodiments, an electrowetting-based device may comprise electrodes, wherein said electrodes may comprise dimensions ranging from about 100 µm by 100 µm to about 10 cm by 10 cm. In some embodiments, an electrowetting-based device may comprise electrodes of any shape and dimension. For example, said electrodes may comprise, but are not limited to comprising, rectangular, circular, triangular, trapezoidal, and/or irregular electrodes shapes. In some embodiments, an electrowetting-based device may comprise an electrode shape as presented in **FIG. 4A-4F**. In some embodiments, an electrowetting-based device may comprise electrodes, wherein adjacent electrodes may be interdigitated with one another, e.g., **FIG. 4D****-****FIG. 4F****.** In some embodiments, an electrowetting-based device may comprise electrodes, wherein adjacent electrodes may not be interdigitated with one another. In some embodiments, an electrowetting-based device may comprise electrodes, wherein said electrodes may comprise indium tin oxide ("ITO"). In some embodiments, an electrowetting-based device may comprise electrodes, wherein said electrodes may comprise ITO, transparent conductive oxides ("TCOs"), conductive polymers, carbon nanotubes ("CNT"), graphene, nanowire meshes and/or ultra thin metal films. In some embodiments, an electrowetting-based device may comprise electrodes, wherein said electrodes may comprise any transparent electrical-conducting material. In some embodiments, an electrowetting-based device may comprise a gap between the top plate and the bottom plate such that the volume of a droplet may be a desired volume, e.g., the spacing between the top plate and the bottom plate may be 0.5 mm which may result in a droplet volume of about 12.5 µl in volume. In some embodiments, the electrowetting-based device may comprise droplets that are about 1 µl or less, about 1 µl or more, about 2 µl or more, about 3 µl or more, about 4 µl or more, about 5 µl or more, about 10 µl or more, about 12.5 µl or more, about 15 µl or more, or about 20 µl or more in volume. In some embodiments, the electrowetting-based device may comprise droplets that are about 12.5 µl in volume. In some embodiments, the electrowetting-based device may comprise droplets that are about 1 picoliter to about 5 mL in volume. In some embodiments, an electrowetting-based device may comprise a heating element, e.g., an inductive heating element. In some embodiments, an electrowetting-based device may comprise a plurality of inlet/outlet ports for loading and removal of different samples or of the same sample and for introduction and removal of filler fluid(s), e.g., eight inlet/outlet ports for loading and removal of eight different samples and two dedicated inlet/outlet ports for filler fluid(s). In some embodiments, an electrowetting-based device may comprise a single inlet/outlet port for loading and removal of the same sample or of different samples and for the introduction and removal of filler fluid(s). In some embodiments, an electrowetting-based device may comprise between 1 to about 400 inlet/outlet ports for loading and removal of the same sample or of different samples, and/or said device may further comprise between 1 to about 100 inlet/out ports for the introduction and removal of filler fluid(s). In some embodiments, the pitch between two adjacent sample inlet/outlet ports may be such that each port may be loaded with a sample by a multichannel manual pipette or a liquid handling robot-assisted pipetting method, e.g., the pitch between two adjacent sample inlet/outlet ports may be 9 mm. In some embodiments, the pitch between two adjacent sample inlet/out ports may be about 9 mm. In some embodiments, the pitch between two adjacent sample inlet/out ports may range from about 5 mm to about 500 mm.

The term "electrowetting-mediated droplet manipulations" refers to the use of electrowetting to move droplets within the context of the electrowetting device described herein, e.g., using electrical means of affecting hydrophobicity of a surface and a droplet to induce movement of said droplet across said surface. Examples of manipulations include but are not limited to moving droplets, mixing droplets, splitting droplets, and merging droplets.

The term "primer extension target enrichment" ("PETE") refers to a method of target nucleic acid enrichment of the following general description. PETE generally involves the following steps: (a) providing a double stranded oligonucleotide probe having a primer sequence at each 5' end, wherein one strand includes a region that is complementary to the target nucleic acid sequence and includes a primer sequence having retrievable label and a phosphorothioate cap at its 5' end, and wherein the primer sequence at the 5' end of the other strand is phosphorylated; (b) amplifying the probe using an amplification reaction, e.g. PCR, rolling circle amplification, etc.; (c) cleaving the strand having the 5' phosphorylation to generate a single stranded probe having a 5' retrievable label (d) hybridizing the single stranded probe having a 5' retrievable label to a target nucleic acid sequence of interest; (e) enriching the target nucleic acid sequence of interest by binding the 5' retrievable label of the hybridized, single stranded probe having a 5' retrievable label to a substrate; and (f) releasing the nucleic acid sequence of interest from the substrate by denaturing. The retrievable label may be a biotin, for example. Additionally, the step of enriching may be performed by binding the label to streptavidin attached to a substrate, e.g., the substrate may be a magnetic bead. Also, an exonuclease, such as lambda exonuclease or another exonuclease, may be used to cleave the strand having the 5' phosphorylation.

The term "library" generally refers to a collection of nucleic acid fragments, and in particular DNA fragments. Libraries may include but are not limited to cDNA libraries, e.g., libraries formed from reverse-transcribed RNA, genomic libraries, e.g., libraries formed from genomic DNA derived from an organism, and randomized mutant libraries, e.g., libraries formed by *de novo* gene synthesis where alternative nucleotides or codons are incorporated. cDNA libraries are often useful for transcriptome analysis of an organism. Nucleic acid sources for libraries include, but are not limited to, single cells, heterogenous populations of cells, and biological samples, for example.

The terms "adapter", "adapter ligand", and "adapter oligonucleotide" refer to specific oligonucleotides that are ligated to nucleic acid fragments, generally DNA fragments, for future DNA sequencing processes.

The term "end repair" refers to the process that ensures that each nucleic acid molecule, generally in the form of DNA, is free of overhangs, and contains 5' phosphate and 3' hydroxyl groups as nucleic acid fragmentation, in particular DNA fragmentation, generally does not result in homogeneous, blunt-ended fragments.

The term "ligation" generally refers to covalently joining the ends of two nucleic acid fragments. An exemplary method of ligation may be a method whereby the 5' and 3' ends of two nucleic acid fragments are joined through use of an enzyme.

The term "adapter ligation" refers to any means by which an adapter may be ligated to a nucleic acid fragment. Adapter ligation may occur through any ligation technique known in the art, such as blunt-ended ligation, A-tailing ligation, or single-stranded splint ligation, for example.

The term "single stranded splint ligation" generally refers to a process by which a DNA ligase catalyzes the ligation of adjacent, single-stranded DNA splinted by a complementary RNA strand.

The terms "blunt end ligation" and "blunt ended ligation" generally refer to the process by which DNA fragments that are blunt-ended, i.e., not containing overhangs, said overhangs generally being complementary, are joined together.

The term "sticky end ligation" generally refers to the process by which DNA overhangs that are generally complementary to one another are joined together.

The term "TA ligation" generally refers to a process by which DNA fragments may be joined that entails the following description. PCR-based amplification usually generates blunt-ended PCR products, but PCR using a *Taq* polymerase, for example, can add an extra adenine to the 3' end of a PCR product. This property may be exploited in TA ligation wherein the ends of the PCR product can anneal to the T end of another DNA fragment. TA ligation is therefore a form of sticky end ligation. In some instances, blunt-ended vectors may be turned into a vector for TA ligation with dideoxythymidine triphosphate ("ddTTP") using terminal transferase, for example.

The terms "A-tailing ligation" and "dA-tailing ligation" refer to incorporation of non-templated deoxyadenosine 5'-monophosphate (dAMP) onto the 3' end of blunted DNA fragments. dA-tails prevent concatamer formation during downstream ligation steps and enable DNA fragments to be ligated to adapters with complementary dT-overhangs

The terms "nucleic acid sequencing reaction" and "next generation sequencing reaction" ("NGS") generally refer to a process of determining the precise order of nucleotides within a nucleic acid molecule. Examples of sequencing techniques include but are not limited to Helicos True Single Molecule Sequencing, single molecule, real-time ("SMRT") technology of Pacific Biosciences, chemical-sensitive field effect transistor ("chemFET") array to sequence DNA (for example, as described in US Patent Application Publication No. 20090026082), DNA nanoball sequencing, Massively Parallel Signature Sequencing (MPSS), Polony sequencing, Solexa sequencing, ILLUMINA^{™}-based sequencing platforms and methods, SOLiD technology, and Ion Torrent^{™} sequencing, for example.

A sequencing technique that can be used with the methods of the provided invention includes, for example, Helicos True Single Molecule Sequencing (tSMS) (Harris T. D. et al. (2008) Science 320: 106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm. The flow cell is then loaded into an instrument, e.g., HeliScope sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step. Further description of tSMS is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Another example of a sequencing technology that may be used with the methods of the provided invention includes the SMRT technology of Pacific Biosciences to sequence both DNA and RNA. In SMRT, each of the four DNA bases is attached to one of four different fluorescent dyes. These dyes are phospho-linked. A single DNA polymerase is immobilized with a single molecule of template single stranded DNA at the bottom of a zeromode waveguide ("ZMW"). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated. In order to sequence RNA, the DNA polymerase is replaced with a reverse transcriptase in the ZMW, and the process is followed accordingly.

Another example of a sequencing technique that can be used with the methods of the provided invention involves using a chemFET array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used with the methods of the provided invention involves using an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

DNA nanoball sequencing is a type of high throughput sequencing technology used to determine the entire genomic sequence of an organism. The method uses rolling circle replication to amplify small fragments of genomic DNA into DNA nanoballs. Unchained sequencing by ligation is then used to determine the nucleotide sequence. This method of DNA sequencing allows large numbers of DNA nanoballs to be sequenced per run. See WO2014122548 and Drmanac et al., Science. 2010 Jan 1;327(5961):78-81; Porreca, Nat Biotechnol. 2010 Jan;28(1):43-4.

Massively Parallel Signature Sequencing (MPSS) was one of the earlier nextgeneration sequencing technologies. MPSS uses a complex approach of adapter ligation followed by adapter decoding, reading the sequence in increments of four nucleotides.

Polony sequencing combines an in vitro paired-tag library with emulsion PCR, an automated microscope, and ligation-based sequencing chemistry to sequence an *E. coli* genome. The technology was also incorporated into the Applied Biosystems SOLiD platform.

In Solexa sequencing, DNA molecules and primers are first attached on a slide and amplified with polymerase so that local clonal colonies, initially coined "DNA colonies", are formed. To determine the sequence, four types of reversible terminator bases (RT -bases) are added and non-incorporated nucleotides are washed away. Unlike pyrosequencing, the DNA chains are extended one nucleotide at a time and image acquisition can be performed at a delayed moment, allowing for large arrays of DNA colonies to be captured by sequential images taken from a single camera.

SOLiD technology employs sequencing by ligation. Here, a pool of all possible oligonucleotides of a fixed length is labeled according to the sequenced position.

Oligonucleotides are annealed and ligated; the preferential ligation by DNA ligase for matching sequences results in a signal informative of the nucleotide at that position. Before sequencing, the DNA is amplified by emulsion PCR. The resulting beads, each containing single copies of the same DNA molecule, are deposited on a glass slide. The result is sequences of quantities and lengths comparable to Solexa sequencing.

In Ion Torrent^{™} sequencing, DNA is sheared into fragments of approximately 300-800 base pairs, and the fragments are blunt ended. Oligonucleotide adapters are then ligated to the ends of the fragments. The adapters serve as primers for amplification and sequencing of the fragments. The fragments can be attached to a surface and is attached at a resolution such that the fragments are individually resolvable. Addition of one or more nucleotides releases a proton (H+), which signal detected and recorded in a sequencing instrument. The signal strength is proportional to the number of nucleotides incorporated. Ion Torrent data may also be output as a FASTQ file. See U.S. publication numbers 2009/0026082, 2009/0127589, 2010/0035252, 2010/0137143, 2010/0188073, 2010/0197507, 2010/0282617, 2010/0300559, 2010/0300895, 2010/0301398, and 2010/0304982.

The term "sample" as used herein includes a specimen or culture (e.g., microbiological cultures) that includes nucleic acids and/or a target nucleic acid. The term "sample" is also meant to include both biological and environmental samples. A sample may include a specimen of synthetic origin. A "biological sample" may include, but is not limited, to whole blood, serum, plasma, umbilical cord blood, chorionic villi, amniotic fluid, cerebrospinal fluid, spinal fluid, lavage fluid (e.g., bronchioalveolar, gastric, peritoneal, ductal, ear, arthroscopic), biopsy sample, urine, feces, sputum, saliva, nasal mucous, prostate fluid, semen, lymphatic fluid, bile, tears, sweat, breast milk, breast fluid, embryonic cells and fetal cells. The biological sample may be blood, and may be plasma. As used herein, the term "blood" encompasses whole blood or any fractions of blood, such as serum and plasma as conventionally defined. Blood plasma refers to the fraction of whole blood resulting from centrifugation of blood treated with anticoagulants. Blood serum refers to the watery portion of fluid remaining after a blood sample has coagulated. Environmental samples include environmental material such as surface matter, soil, water and industrial samples, as well as samples obtained from food and dairy processing instruments, apparatus, equipment, utensils, disposable and non-disposable items.

The term "analysis" generally refers to a process or step involving physical, chemical, biochemical, or biological analysis that includes characterization, testing, measurement, optimization, separation, synthesis, addition, filtration, dissolution, or mixing.

The term "chemical" refers to a substance, compound, mixture, solution, emulsion, dispersion, molecule, ion, dimer, macromolecule such as a polymer or protein, biomolecule, precipitate, crystal, chemical moiety or group, particle, nanoparticle, reagent, reaction product, solvent, or fluid any one of which may exist in the solid, liquid, or gaseous state, and which is typically the subject of an analysis.

The term "protein" generally refers to a set of amino acids linked together usually in a specific sequence. A protein can be either naturally-occurring or man-made. As used herein, the term "protein" includes amino acid sequences that have been modified to contain moieties or groups such as sugars, polymers, metalloorganic groups, fluorescent or light-emitting groups, moieties or groups that enhance or participate in a process such as intramolecular or intermolecular electron transfer, moieties or groups that facilitate or induce a protein into assuming a particular conformation or series of conformations, moieties or groups that hinder or inhibit a protein from assuming a particular conformation or series of conformations, moieties or groups that induce, enhance, or inhibit protein folding, or other moieties or groups that are incorporated into the amino acid sequence and that are intended to modify the sequence's chemical, biochemical, or biological properties. As used herein, a protein includes, but is not limited to, enzymes, structural elements, antibodies, hormones, electron carriers, and other macromolecules that are involved in processes such as cellular processes or activities. Proteins typically have up to four structural levels that include primary, secondary, tertiary, and quaternary structures.

A "probe" is generally defined as a nucleic acid capable of binding to a target nucleic acid of complementary sequence through one or more types of chemical bonds, usually through complementary base pairing, usually through hydrogen bond formation, thus forming a duplex structure. The probe binds or hybridizes to a "probe binding site." The probe can be labeled with a detectable label to permit facile detection of the probe, particularly once the probe has hybridized to its complementary target. The label attached to the probe can include any of a variety of different labels known in the art that can be detected by chemical or physical means, for example. Suitable labels that can be attached to probes include, but are not limited to, radioisotopes, fluorophores, chromophores, mass labels, electron dense particles, magnetic particles, spin labels, molecules that emit chemiluminescence, electrochemically active molecules, enzymes, cofactors, and enzyme substrates. Probes can vary significantly in size. Some probes are relatively short. Generally, probes are at least 7 to 15 nucleotides in length. Other probes are at least 20, 30 or 40 nucleotides long. Still other probes are somewhat longer, being at least 50, 60, 70, 80, 90 nucleotides long. Yet other probes are longer still, and are at least 100, 150, 200 or more nucleotides long. Probes can be of any specific length that falls within the foregoing ranges as well.

For purposes of the present disclosure, it will be understood that when a given component such as a layer, region, liquid or substrate is referred to herein as being disposed or formed "on", "in" or "at" another component, that given component can be directly on the other component or, alternatively, intervening components (e.g., one or more buffer layers, interlayers, electrodes or contacts) can also be present. It will be further understood that the terms "disposed on" and "formed on" are used interchangeably to describe how a given component is positioned or situated in relation to another component. Hence, the terms "disposed on" and "formed on" are not intended to introduce any limitations relating particular methods of material transport, deposition, or fabrication.

The term "communicate" is used herein to indicate a structural, functional, mechanical, electrical, optical, thermal, or fluidic relation, or any combination thereof, between two or more components or elements. As such, the fact that one component is said to communicate with a second component is not intended to exclude the possibility that additional components may be present between, and/or operatively associated or engaged with, the first and the second component.

### AN ELECTROWETTING-BASED DEVICE FOR AUTOMATED qPCR

qPCR has become established as a standard method for nucleic acid quantification in all areas of molecular biology. The method generally involves the amplification of a target nucleic acid using an amplification reaction, such as PCR, and the product of the amplification is monitored in real time through the use of a detection agent. It is a quantitative amplification reaction, meaning that the concentration (relative or absolute) of the amplified target nucleic acid can be determined. Conversely, in conventional PCR only the end-result of amplification can only be measured after the PCR is completed (end-point detection), and overamplification can often lead to mutation bias.

Though performing a qPCR reaction using conventional protocols can help to avoid issues associated with overamplification, performing an off-line qPCR using conventional formats can be time consuming and may require larger than desired quantities of what is often a limited starting amount of target nucleic acid. Conventionally, a sample containing the target nucleic acid is used for conventional format qPCR to determine the appropriate number of cycles needed to obtain the desired concentration of amplified target nucleic acid. Following determination of the number of cycles needed for optimal target nucleic acid amplification, end-point detection-based qPCR is performed on a second sample containing the target nucleic acid to be amplified. Generally, the reactions for both the first and second samples may be as large as tens of microliters each, which often represents a large volume of precious target nucleic acid starting samples. Additionally, conventional qPCR uses a wellbased format, which often makes recovery of individual reactions difficult. As described herein, an electrowetting-based device presents a useful alternative platform for qPCR-based assays as well as any form of target nucleic acid amplification wherein quantitation of the target nucleic acid is desired or the extent of product accumulation is to be measured during amplification. Generally, a droplet or multiple droplets comprising a target nucleic acid and the necessary components to perform and to quantitate an amplification reaction are provided on an electrowetting-based device, and said droplet or multiple droplets will often be a nanoliter or less in total volume each, thereby allowing for vast reagent savings, particularly of precious target nucleic acid starting samples. Additionally, two or more samples can be amplified in parallel using an electrowetting-based device. For example, the conventional qPCR procedure described above comprising two samples for two separate reactions, wherein one reaction is used to determine the number of amplification cycles necessary to generate the desired concentration of amplified target nucleic acid, and one reaction is used to apply the pre-determined number of amplification cycles to the second sample, can occur simultaneously on the electrowetting-based device as any of the reaction droplets may be quantitated and may be recovered after any amplification cycle.

Since qPCR is a very sensitive technique, and the dynamic range of this assay extends to very low template copy numbers, the reliability of results is highly dependent on accurate liquid handling. An electrowetting-based device as described herein allows the performance of highly accurate liquid manipulations and metering through electrowetting-mediated droplet manipulations, thereby decreasing the chance for liquid handling-related amplification inaccuracies and increasing the accuracy of quantitative measurements.

A method of using the electrowetting-based device described herein generally relates to its use for effecting amplification of a target nucleic acid. In general, electrowetting-mediated droplet manipulations can allow for the creation of droplets and/or subsets of droplets containing all of the necessary components for performing an amplification reaction from starting droplets that contain any combination of the components necessary for the reaction. For example, droplets comprising a target nucleic acid may be provided on the device, and/or a droplet comprising a target nucleic acid may be formed on the device through using electrowetting-mediated droplet manipulations. Through electrowetting-mediated droplet manipulations, smaller droplets may be formed from the initial droplet comprising said target nucleic acid. A separate droplet or droplets containing a reaction mixture, or components thereof, that are necessary to perform an amplification reaction may also be provided on the device. Electrowetting-mediated droplet manipulations can allow for the creation of smaller volume droplets from the initial starting droplet, and further electrowetting-mediated droplet manipulations can be used to merge and mix a droplet comprising said reaction mixture with said target nucleic acid containing droplet. A detection agent may be provided in a droplet on the electrowetting-based device, and using a similar process as described above, smaller volume droplets comprising said detection agent may be merged and mixed with a droplet comprising said nucleic acid and/or said reaction mixture. Alternatively, the target nucleic acid, reaction mixture, and detection agent may be provided on the device as a single droplet, and many smaller volume droplets may be created from the initial droplet using electrowetting-mediated droplet manipulations. In an embodiment of the invention, electrowetting-mediated droplet manipulations can be used to create two subsets of droplets for a subsequent amplification reaction. Both subsets of droplets may comprise all of the components necessary to perform an amplification reaction, including a target nucleic acid, however, one subset of droplets may contain a detection agent and one subset of droplets may not contain a detection agent. In other embodiments of the invention, multiple subsets of droplets may each contain the same or a different target nucleic acid and/or the same or a different detection agent.

Following generation of droplets or subsets of droplets containing the components necessary to perform an amplification reaction, amplification of the target nucleic acid may occur on the electrowetting-based device. Amplification may occur through such methods as standard PCR thermocycling, isothermal amplification, hot start amplification, or any method that will result in amplification of a target nucleic acid. In order to effect a temperature change that may be required for a desired method of amplification, droplets comprising the target nucleic acid may be moved using electrowetting-mediated droplet manipulations, e.g., using electrical means of affecting hydrophobicity of a surface and a droplet to induce movement of said droplet across said surface, to a portion of the device that can be heated through the use of heating elements. Said heating elements may comprise a heater on the chip or a heater separate from the chip. For example, heating can be achieved through using contact heaters that are a separate element from the device itself, or through heating elements that are fabricated as a part of the device. Also, heating can be achieved through using inductive heating elements that are a separate element from the device itself, or through inductive heating elements that are fabricated as a part of the device. Through use of any of said heating elements, the droplets may be thermocycled as is necessary for the desired method of amplification. Due to the efficient heat transfer properties that are present when performing thermocycling on an electrowetting-based device, a vast savings in the time necessary to amplify a target nucleic acid is achieved. For example, the thermal cycling steps generally associated with PCR-based amplification, e.g. temperatures designed to result in the general thermocycle steps of melting, annealing, and extension, may be effected through use of an inductive heating elements present within the electrowetting-based device. Due to the high efficiency of heat transfer, 30 cycles of a three step thermocycle may occur in as little as ten minutes. In order to effect hot start amplification on the electrowetting-based device, one or more components of the PCR may be kept separate from each other by partitioning the various components into different droplets or different subsets of droplets. For example, this type of methodology can be accomplished by partitioning a mixture containing primers, template, nucleotides, and water into a first droplet or first subset of droplets, then partitioning the remaining PCR components, such as a nucleic acid polymerase, buffer, and MgCl₂, into a second droplet or subset of droplets. Said partitioning may be accomplished through use of electrowetting-mediated droplet manipulations, for example. Both the first and second droplets, or first and second subsets of droplets, may then be heated in parallel through use of heating elements, and then said first and second droplets or subsets of droplets may be merged and mixed after the desired temperature has been attained.

Amplification and quantitation of a target nucleic acid within droplets provided or formed through electrowetting-mediated droplet manipulations on an electrowetting-based device may generally proceed whereby all droplets provided or formed on the device contain an agent for nucleic acid detection. Said droplets may comprise subsets of droplets wherein each subset of droplets comprises a different target nucleic acid for subsequent amplification, or said droplets may all comprise the same target nucleic acid for subsequent amplification. Conversely, two subsets of droplets may be provided or formed on the device, wherein one subset of droplets contains an agent for target nucleic acid detection and wherein the other subset of droplets does not contain an agent for target nucleic acid detection. The conditions necessary to effect nucleic acid amplification, based upon the desired method of amplification, are created through use of electrowetting-mediated droplet manipulations and of the heating elements present in the device. Said conditions can be applied to all droplets and/or subsets of droplets in parallel, regardless of the absolute number of droplets or the presence or absence of distinct subsets of droplets. As amplification of the target nucleic acid proceeds, the amount of target nucleic acid generated may be measured at various stages of the amplification cycle, preferably at the end of each cycle of amplification cycle, through measurement and quantification of a detection agent present within some or all of the droplets and/or subsets of droplet. Droplets may be moved to a detection area through electrowetting-mediated droplet manipulations, and the concentration of a target nucleic acid measured through use of a detection agent. Alternatively, the electrowetting-based device may be capable having a dynamic detection area, e.g., a scanning head for detecting the signal of a detection agent, whereby the quantity of target nucleic acid within a droplet can be measured at various locations throughout the chip through detection of a detection agent.

In the case wherein at least two subsets of droplets are provided on the electrowetting-based device, one subset of droplets not containing an agent for target nucleic acid detection and at least one subset of droplet containing an agent for target nucleic acid detection, once a desired amount of nucleic acid has been obtained as determined through quantification of the detection agent in the subset of droplets containing the detection agent, the subset of droplets not containing a detection agent may be recovered from the device through electrowetting-mediated droplet manipulations for further downstream processing steps. If so desired, the subset of droplets containing the detection agent may be recovered from the device as well for any future downstream processing and/or assays. Both subsets of droplets should contain an equivalent concentration of target nucleic acid as both subsets of droplets underwent amplification in parallel. Wherein all droplets contain an agent for detection, any amount or combination of individual droplets may be manipulated using the electrowetting-based device in order to recover each of said droplets once the amount of target nucleic acid amplified has reached the desired concentration. If desired, any or all of said droplets or subsets of droplets can be mixed with other reagents and/or reaction mixtures to prepare the droplets for subsequent processing, e.g., nucleic acid sequencing reactions.

The amplified target nucleic acid resulting from the qPCR amplification using an electrowetting-based device as described herein may be used for such downstream applications as, but not limited to, NGS sequencing using various NGS sequencing platforms, whole-genome shotgun sequencing, whole exome or targeted sequencing, amplicon sequencing, mate pair sequencing, RIP-seq/CLIP-seq, ChIP-seq, RNA-seq (generally starting with cDNA), and/or methyl-seq, for example, following recovery of the droplets containing the desired amount of amplified target nucleic acid. The electrowetting device described herein may additionally be used for processes such as general amplification-based processes including amplicon generation and target enrichment in addition to various applications during different stages of library amplification and quantitation. Target nucleic acid enrichment may occur through primer extension target enrichment ("PETE"), wherein PETE may be performed using the electrowetting-based device described herein. PETE generally involves the following steps: (a) providing a double stranded oligonucleotide probe having a primer sequence at each 5' end, wherein one strand includes a region that is complementary to the target nucleic acid sequence and includes a primer sequence having retrievable label and a phosphorothioate cap at its 5' end, and wherein the primer sequence at the 5' end of the other strand is phosphorylated; (b) amplifying the probe using an amplification reaction, e.g. PCR, rolling circle amplification, etc.; (c) cleaving the strand having the 5' phosphorylation to generate a single stranded probe having a 5' retrievable label; (d) hybridizing the single stranded probe having a 5' retrievable label to a target nucleic acid sequence of interest; (e) enriching the target nucleic acid sequence of interest by binding the 5' retrievable label of the hybridized, single stranded probe having a 5' retrievable label to a substrate; and (f) releasing the nucleic acid sequence of interest from the substrate by denaturing. The process described above may be performed within the context of droplets provided on the electrowetting-based device comprising the necessary reaction elements, and wherein the above steps are accomplished through use of electrowetting-based droplet manipulations and through the use of a detection zone and/or, if necessary, a heating element associated with the electrowetting-based device.

Library preparation for the next generation sequencing generally involves the ligation of specific adapter oligonucleotides to fragments of nucleic acid to be sequenced. Ligation of adapters may occur through various methods known in the art, such as single-stranded splint ligation, blunt-ended adapter ligation, sticky end ligation, TA-ligation, and/or dA-tailing ligation of adapters. When the beginning target nucleic acid is DNA, first, the DNA is fragmented to the optimal length determined by the downstream NGS sequencing method. DNA fragmentation generally does not result in homogeneous, blunt-ended fragments, so end repair is needed to ensure that each molecule is free of overhangs, and contains 5' phosphate and 3' hydroxyl groups. Adapter ligation may then occur using such methods as single-stranded splint ligation, blunt-ended adapter ligation, sticky end ligation, TA-ligation, and/or dA-tailing ligation of adapters. Libraries to be used in blunt-ended adapter ligation, including Ion Torrent^{™} or SOLiD^{™} 4 library construction, can be used directly in the ligation step. For Illumina^{®} libraries and some libraries intended for the 454^{™} platform, incorporation of a non-templated deoxyadenosine 5'-monophosphate (dAMP) onto the 3' end of blunted DNA fragments, a process known as dA-tailing, may be necessary. dA-tails prevent concatamer formation during downstream ligation steps, and enable DNA fragments to be ligated to adapters with complementary dT-overhangs. The desired adapter ligated DNA size for Illumina, SOLiD and Ion Torrent platforms can be selected via gel electrophoresis before amplification following any method of adapter ligation.

In an embodiment of the invention, an electrowetting-based device may be used to quantitate the number of adapter-ligated target nucleic acid molecules at various steps of library preparation, wherein the library is intended for use with a next generation sequencing ("NGS") platform. Any of the below-described methods and reactions may be performed using droplets comprising the necessary reaction components wherein the droplets are provided on an electrowetting-based device and electrowetting-based droplet manipulations combined with the electrowetting-based device allow for the desired reaction and analysis to occur. qPCR has demonstrated usefulness as a method for amplification and quantitation of nucleic acid libraries prior to any nucleic acid sequencing, in particular those methods using a NGS platform or technique. In general, the primary steps in preparing nucleic acids for NGS analysis are as follows: (a) fragmenting and/or sizing the target nucleic acid sequences to a desired length; (b) converting the target nucleic acid to double-stranded DNA; (c) attaching oligonucleotide adapters to the ends of target fragments; and (d) quantitating the final library product for sequencing, generally through the process of qPCR. More specifically, steps that are normally used for library preparation are as follows: (a) fragmentation of the nucleic acid source material; (b) end repair; (c) phosphorylation of the 5' ends; (d) A-tailing of the 3' ends to facilitate ligation to sequencing adapters; (e) ligation of adapters; and (f) some number of PCR cycles to enrich for product that has adapters ligated to both ends. qPCR using the electrowetting-based device described herein may allow for quantification of the number of adapter-ligated molecules in a library prepared for NGS at various stages of sequencing workflows, including: (a) after adapter ligation to determine the amount of input material converted to adapter-ligated molecules (conversion rate) and/or the quantity of template used for library amplification; (b) after library amplification, to determine whether a sufficient amount of each library has been generated and/or to ensure equal representation of indexed libraries pooled for target capture or cluster amplification; and/or (c) prior to cluster amplification, to confirm that individual libraries or sample pools are diluted to the optimal concentration for NGS flow cell loading, as overestimation or underestimation of library concentration may result in suboptimal utilization of sequencing capacity. qPCR may be used after the post-ligation cleanup steps (prior to library amplification) to provide useful data for optimization. Quantification at this stage allows a user to assess the efficiency of the core library construction process (end repair, A-tailing, and ligation) by determining the percentage of input DNA converted to adapter-ligated molecules, as well as library amplification with the selected number of cycles, based on the actual amount of template DNA used in the PCR amplification reaction. Additionally, if size selection is performed at any stage, qPCR quantification before and after size selection may also be helpful to define the relative benefit of size selection, and to determine the loss of material associated with the process.

An aspect of the invention generally relates to a method of using the electrowetting-based device described herein to avoid library overamplification bias as may occur during target nucleic acid amplification. Excessive library amplification can result in other unwanted artifacts such as amplification bias, PCR duplicates, chimeric library inserts, and nucleotide substitutions. The extent of library amplification should therefore be limited as much as possible. The ability to amplify droplets comprising a target nucleic acid in parallel while monitoring the amount of target nucleic acid present within any of said droplets allows for determination of the ideal amplification parameters and number of amplification cycles necessary to obtain the desired amount of target nucleic acid. Additionally, if desired, a sample of target nucleic acid may be subject to a qPCR reaction using a standard qPCR-based approach in order to determine the optimal number of amplification cycles necessary to obtain the desired concentration of target nucleic acid. Once the number of cycles has been determined, said number of cycles may be applied to performing a qPCR reaction on the electrowetting-based device described herein.

Any desired target nucleic acid may be used as a template for qPCR on an electrowetting-based device. For example, RNA can be used as a template (e.g. in case of gene expression studies or detection of RNA viruses) for qPCR using an electrowetting-based device as described herein. In this case the RNA needs to be reverse transcribed into DNA (also termed complementary DNA or cDNA) before it is amplified with qPCR. The term for this combined method is "real-time reverse transcription PCR" ("qRT-PCR" or "RT-qPCR"). Additionally, the electrowetting-based device may be used to effect transcriptome-based analyses, in particular originating from the mRNA from a single cell.

The electrowetting-based device described herein may be used for amplification of a starting concentration of a single copy of a target nucleic acid. If desired, a droplet may be provided on the electrowetting-based device that contains a concentration of target nucleic acid greater than a single copy. Electrowetting-mediated droplet operations may be used to split the droplet until a single copy, or, on average, less than a single copy, of the target nucleic acid is present in each droplet. Said droplet containing one or on average less than one copy of the target nucleic acid may then be merged and mixed with a droplet containing the reagents necessary to perform an amplification reaction and to detect the quantity of nucleic acid produced as a result of the amplification reaction. Said detection may occur within the context of two subsets of droplets, both of which contain on average less than one copy of target nucleic acid per droplet, but wherein only one subset of droplets contains a detection agent.

The electrowetting-based device described herein may also be used to perform a melt curve analysis. A melt curve analysis is typically included at the end of SYBR^{®} Green I-based qPCR assays to provide information about the specificity of the reaction. Melt curve analysis has been found useful for the identification of carry-over adapter-dimer in ILLUMINA^{™} libraries. Adapter-dimers, formed during library construction with full-length adapters, are very efficiently amplified, and will lead to an overestimation of library concentration if present in significant quantities.

The electrowetting-based device described herein is capable of performing fully automated qPCR reactions with droplets provided on the device. Using a computer that is in communication with the electrowetting-based device and all components associated with the electrowetting-based device, e.g., the heating elements and detection zones, programs that were written beforehand can be executed to perform all necessary electrowetting-mediated droplet manipulations, amplification reaction procedures, target nucleic acid quantitation measurements effected through detection agent detection at a detection zone, and subsequent recovery of any and/or all droplets that contain the desired outcome of the amplification reaction. Following recovery, the desired product of the target nucleic acid amplification reaction may be subject to further assays as desired by the user. In order to perform the desired on-device reaction, a user may need to simply input the sample that contains the target nucleic acid under investigation into the device. Once on the electrowetting-based device, automated protocols can be executed that can detect the starting quantity of target nucleic acid present, and subsequently that can perform the electrowetting-mediated droplet manipulations necessary to generate droplets and/or subsets of droplets that contain the desired concentration of target nucleic acid along with any other desired reaction components and detection agents. Based upon the input conditions, protocols can be executed to obtain the desired amount of target nucleic acid amplification, and the droplets containing the desired amount of target nucleic acid may further be recovered from the device for further processing steps, e.g., sequencing reactions.

In general, use of an electrowetting-based device for performing target nucleic acid amplification and subsequent quantification may greatly increase the speed, efficiency, and sensitivity with which biomarkers in the form of nucleic acids may be detected. In particular, said device may represent a powerful point-of-care diagnostics tool, allowing for the detection of biomarkers that represent the presence of a disease in a patient from less than one microliter of biological sample, and/or for generation of target nucleic acid libraries for subsequent sequencing reactions.

In some embodiments, the electrowetting-based device described herein may be used to perform an amplification method, e.g., an automated amplification method, e.g., an amplification reaction, wherein said amplification method comprises the use of KAPA HiFi DNA polymerase. In some embodiments, the electrowetting-based device described herein may be used to perform an amplification method, wherein said amplification method comprises the use of a polymerase designed for use with SYBR green I chemistry, e.g., KAPA SYBR FAST DNA polymerase. In some embodiments, the electrowetting-based device described herein may be used to perform an amplification method, e.g., an automated amplification method, e.g., an amplification reaction, wherein said amplification method comprises the use of KAPA SYBR FAST DNA polymerase. In some embodiments, the electrowetting-based device described herein may be used to perform an amplification method, wherein said amplification method comprises the use of intercalating dyes, e.g., SYBR green I, SYTO9, OPD, and/or Roche LIGHTCYCLER^{®} 480 ResoLight Dye. Said intercalating dyes may comprise a concentration ranging from about 1 pM to about 1 µM, e.g., about 1 pM, about 10 pM, about 100 pM, about 1 nM, about 10 nM, about 100 nM, or about 1 µM. In some embodiments, said intercalating dye may comprise a redox probe, e.g., OPD. Said redox probe, e.g., OPD, may comprise a concentration ranging from about 1 pM to about 1 µM, e.g., about 1 pM, about 10 pM, about 100 pM, about 1 nM, about 10 nM, about 100 nM, or about 1 µM. In some embodiments, the electrowetting-based device described herein may be used to perform an amplification method, e.g., an automated amplification method, e.g., an amplification reaction, wherein said amplification method comprises the use of thermocycles wherein temperatures of said thermocycles range from about 50°C to about 98°C, e.g., about 50°C, about 60°C, about 65°C, about 72°C, about 95°C, or about 98°C. In some embodiments, the electrowetting-based device described herein may be used to perform an amplification method, e.g., an automated amplification method, e.g., an amplification reaction, wherein said amplification method comprises the reaction times, wherein said reaction time may comprise the amount of time at a single step of a thermocycle, any combination of thermocycle steps, or the amount of time for a complete amplification reaction, ranging between 1 s to about 5 min., e.g., about 1 sec, about 5 sec, about 10 sec, about 20 sec, about 30 sec, about 45 sec, about 1 min, and/or about 5 min.

In some embodiments, the electrowetting-based device described herein may be used to perform an amplification method, e.g., an automated amplification method, e.g., an amplification reaction, wherein said amplification method comprises the use of a master mix, e.g., a mix comprising polymerase, dNTP, MgCl₂, and oligonucleotide primers. Said master mix may comprise dNTP(s), and the concentration of said dNTP(s) may comprise a range from about 1 mM to about 100 mM, e.g., about 1 mM, about 10 mM, or about 100 mM. Said master mix may comprise MgCl₂, and the concentration of MgCl₂ may comprise a range from about 1 mM to about 100 mM, e.g., about 1 mM, about 10 mM, or about 100 mM. Said master mix may comprise oligonucleotide primer(s), and the concentration of oligonucleotide primer(s) may comprise a range from about 1 nM to about 1 mM, e.g., about 1 nM, about 1 µM, or about 1 mM.

In some embodiments, an electrowetting-based device described herein may be used to perform an amplification method, e.g., an automated amplification method, e.g., an amplification reaction, wherein said amplification method comprises the use of filler fluid, e.g., a transparent oil. In some embodiments, said filler fluid may be a transparent oil, e.g., liquid polymerized siloxane, e.g., silicone oil e.g., mineral oil, e.g., paraffin oil. In some embodiments, said filler fluid may comprise liquid polymerized siloxane, silicone oil mineral oil, and/or paraffin oil.

In some embodiments, an electrowetting-based device described herein may be used to perform an amplification method, e.g., an automated amplification method, e.g., an amplification reaction, wherein said amplification method comprises a target concentration of amplified material, e.g., a nucleic acid, e.g., a target nucleic acid, ranging from about 1 pM to about 1 mM, e.g., about 1 pM, about 10 pM, about 100 pM, about 1 nM, about 10 nM, about 100 nM, about 1 µM, about 10 µM, about 100 µM, or about 1 mM. In some embodiments, the electrowetting-based device described herein may be used to perform an amplification method, e.g., an automated amplification method, e.g., an amplification reaction, wherein said amplification method comprises a starting concentration of a nucleic acid, e.g., a target nucleic acid, ranging from about 1 pM to about 1 mM, e.g., about 1 pM, about 10 pM, about 100 pM, about 1 nM, about 10 nM, about 100 nM, about 1 µM, about 10 µM, about 100 µM, or about 1 mM.

### EXAMPLES

As presented in **FIG. 1A-1B****,** droplet reactions are moved between different inductive heating zones using electrowetting-mediated droplet manipulations, and said heating zones are used to perform temperature changes required by a desired amplification reaction method. In **FIG. 1A****,** there is only one droplet reaction for each of four different starting samples (labelled 1-4 in **FIG. 1A**). Each droplet reaction contains a detection agent in the form of an intercalating agent. Any or all droplet reactions are recovered from the device once a desired amount of PCR product has been generated as indicated by fluorescence or electrochemical readings through detection and quantification using a detection agent and the device's detection zone. In **FIG. 1B****,** there are two droplet reactions for each of two different starting samples (labelled 1-2 in **FIG. 1B**), one which contains a detection agent and one which does not. The pairs of droplet reactions are thermocycled in parallel. Any or all of the droplet reactions not containing a detection agent are recovered from the device once a desired amount of PCR product has been generated as indicated by fluorescence or electrochemical readings of the droplet reaction containing the detection agent.

In **FIG. 2A****,** the one half of a pair of droplet reactions for each of eight different starting samples (labelled 1-8 in **FIG. 2A**) is loaded into the device through the sample inlet/outlet port, and is moved to one end of the electrode lane. The eight droplets containing eight different samples of **FIG. 2A** comprise a first subset of droplets. In **FIG. 2B****,** the other half of a pair of droplet reactions for each of said eight different starting samples (labelled another set of 1-8 in **FIG. 2B**) is loaded into the device through the inlet/outlet port, and is moved to the other end of the electrode lane. Said eight different droplets of **FIG. 2B** comprise a second subset of droplets. Each droplet of each subset, e.g., droplet 1 of subset 1 and droplet 1 of subset 2, are identical except that each droplet of the second subset of droplets contains a detection agent in the form of an intercalating agent, whereas each droplet of the first subset of droplets does not contain a detection agent in the form of an intercalating agent. In **FIG. 2C****,** the sixteen droplet reactions are moved between different inductive heating zones using electrowetting-mediated droplet manipulations, and said heating zones are used to perform temperature changes required by a desired amplification reaction method. Any or all droplet reactions are recovered from the device once a desired amount of PCR product has been generated as indicated by fluorescence or electrochemical readings through detection and quantification using a detection agent and the device's detection zone.

FIG. 3 shows a design example of a device for reactions of eight different starting samples, in which each sample is split into two droplets when loaded, an example of which is presented in FIG 2A-C. In **FIG. 3****,** dimensions are indicated in mm, and the "," is a decimal point. In the device, each electrode on which a droplet is placed is designed to be a square with a dimension of 5 by 5 mm, and the gap between top and bottom plate is 0.5 mm, so that the volume of each droplet on an electrically-activated electrode is estimated to be 12.5 µl. In this device, the pitch between two adjacent sample inlet/outlet ports in the top plate is designed to be 9 mm so that eight different samples can be loaded and unloaded in and out of the device through the ports using a multichannel manual pipette or using liquid handling robot-assisted pipetting. Two filler fluid inlet ports are used to fill the device with filler fluid, e.g., low viscosity oils such as a silicone-based oil or a fluorocarbon-based oil. In addition to the square electrode shape presented in **FIG.3****,** additional examples of electrode shape of electrodes of an electrowetting-based device are presented in **FIG.4A-4F****.** Examples of electrode shapes may be squares **(****FIG. 4A****)**, triangles **(****FIG. 4B****)**, trapezoids **(****FIG. 4C****)**, and/or any of the irregular shapes presented in **FIG.4D, FIG. 4E****,** and/or **FIG. 4F****.**

## Claims

1. An automated nucleic acid amplification method which comprises the following steps:
(a) providing an electrowetting-based device;
(b) providing at least two droplets on said electrowetting-based device, wherein each droplet comprises primers that anneal to a target nucleic acid; and a target nucleic acid;
(c) amplifying the target nucleic acid in each said droplet in parallel;
(d) quantitating the amplified target nucleic acid in at least one droplet; and
(e) after a desired amount of the target nucleic acid has been obtained, recovering at least one droplet and using said at least one droplet for further analyzing or processing of said at least one droplet,
**characterized in that**
the analyzing or processing is selected from a group consisting of nucleic acid sequencing, next generation sequencing, whole-genome shotgun sequencing, whole exome sequencing, targeted sequencing, amplicon sequencing, mate pair sequencing, RIP-seq/CLIP-seq, ChIP-seq, RNA-seq, transcriptome sequencing, and methyl-seq.

2. The method of claim 1, wherein:
(i) said droplets each comprise a different target nucleic acid;
(ii) each of said droplets comprises the same target nucleic acid;
(iii) said electrowetting-based device comprises a biplanar configuration of parallel arrays of electrodes to effect electrowetting-mediated droplet manipulations;
(iv) said droplets comprise a mixture of droplets that contain the same target nucleic acid and different target nucleic acids;
(v) the electrowetting-based device comprises a planar configuration of electrodes that effects electrowetting-mediated droplet manipulations;
(vi) said droplets comprise a droplet volume ranging from about 1 picoliter to about 5 mL, optionally wherein said droplets comprise a droplet volume of about 12.5 mL;
(vii) the electrowetting-based device comprises a biplanar configuration of parallel arrays of electrodes or a planar configuration of electrodes to effect electrowetting-mediated droplets manipulations, and wherein said electrodes comprise electrode dimensions ranging from about 100 µm by 100 µm to about 10 cm by 10 cm;
(viii) the electrowetting-based device comprises a biplanar configuration of parallel arrays of electrodes or a planar configuration of electrodes to effect electrowetting-mediated droplets manipulations, and wherein said electrodes are interdigitated;
(ix) the electrowetting-based device comprises a biplanar configuration of parallel arrays of electrodes or a planar configuration of electrodes to effect electrowetting-mediated droplets manipulations, and wherein said electrodes and comprise indium tin oxide ("ITO"), transparent conductive oxides ("TCOs"), conductive polymers, carbon nanotubes ("CNT"), graphene, nanowire meshes and/or ultra thin metal films, optionally wherein said electrodes comprise ITO;
(x) said device comprises inlet/outlet ports wherein the spacing between adjacent ports ranges from about 5 mm to about 500 mm; or
(xi) the electrowetting-based device comprises or is in contact with at least one heating element and at least one detection zone.

3. The method of claim 2, embodiment (v), wherein:
said electrowetting device is in contact with or comprises a heating element and a detection zone; and wherein
(i) said electrowetting-based device comprises square electrodes, optionally wherein said electrodes are about 5 mm by 5 mm; or
(ii) said electrowetting-based device comprises electrodes, wherein said electrodes are square, triangular, rectangular, circular, trapezoidal, and/or irregularly shaped.

## Patentansprüche

1. Automatisiertes Nukleinsäureamplifikationsverfahren, das die folgenden Schritte umfasst:
(a) Bereitstellen einer auf Elektrobenetzung basierenden Vorrichtung;
(b) Bereitstellen von mindestens zwei Tropfen auf der auf Elektrobenetzung basierenden Vorrichtung, wobei jeder Tropfen Primer, die an eine Zielnukleinsäure hybridisieren, und eine Zielnukleinsäure umfasst;
(c) paralleles Amplifizieren der Zielnukleinsäure in jedem Tropfen;
(d) Quantifizieren der amplifizierten Zielnukleinsäure in mindestens einem Tropfen und
(e) nachdem eine gewünschte Menge der Zielnukleinsäure erhalten worden ist, Gewinnen mindestens eines Tropfens und Verwenden des mindestens einen Tropfens für weiteres Analysieren oder Verarbeiten, **dadurch gekennzeichnet, dass** das Analysieren oder Verarbeiten aus einer Gruppe, bestehend aus Nukleinsäuresequenzierung, Sequenzierung der nächsten Generation, Gesamtgenom-Shotgun-Sequenzierung, Gesamtexomsequenzierung, gezielter Sequenzierung, Amplikonsequenzierung, Mate-pair-Sequenzierung, RIP-Seq/CLIP-Seq, ChIP-Seq, RNA-Seq, Transkriptomsequenzierung und Methyl-Seq, ausgewählt ist.

2. Verfahren nach Anspruch 1, wobei:
(i) die Tropfen jeweils eine andere Zielnukleinsäure umfassen;
(ii) jeder der Tropfen dieselbe Zielnukleinsäure umfasst;
(iii) die auf Elektrobenetzung basierende Vorrichtung eine biplanare Konfiguration von parallelen Elektrodenanordnungen umfasst, um durch Elektrobenetzung vermittelte Tropfenmanipulationen zu bewirken;
(iv) die Tropfen ein Gemisch aus Tropfen umfassen, die dieselbe Zielnukleinsäure und verschiedene Zielnukleinsäuren enthalten;
(v) die auf Elektrobenetzung basierende Vorrichtung eine planare Konfiguration von Elektroden umfasst, die durch Elektrobenetzung vermittelte Tropfenmanipulationen bewirkt;
(vi) die Tropfen ein Tropfenvolumen im Bereich von etwa 1 Pikoliter bis etwa 5 ml umfassen, gegebenenfalls wobei die Tropfen ein Tropfenvolumen von etwa 12,5 ml umfassen;
(vii) die auf Elektrobenetzung basierende Vorrichtung eine biplanare Konfiguration von parallelen Elektrodenanordnungen oder eine planare Konfiguration von Elektroden umfasst, um durch Elektrobenetzung vermittelte Tropfenmanipulationen zu bewirken, und wobei die Elektroden Elektrodenausmaße im Bereich von etwa 100 µm mal 100 µm bis etwa 10 cm mal 10 cm umfassen;
(viii) die auf Elektrobenetzung basierende Vorrichtung eine biplanare Konfiguration von parallelen Elektrodenanordnungen oder eine planare Konfiguration von Elektroden umfasst, um durch Elektrobenetzung vermittelte Tropfenmanipulationen zu bewirken, und wobei die Elektroden miteinander verflochten sind;
(ix) die auf Elektrobenetzung basierende Vorrichtung eine biplanare Konfiguration von parallelen Elektrodenanordnungen oder eine planare Konfiguration von Elektroden umfasst, um durch Elektrobenetzung vermittelte Tropfenmanipulationen zu bewirken, und wobei die Elektroden Indiumzinnoxid ("ITO"), transparente leitfähige Oxide ("TCO"), leitfähige Polymere, Kohlenstoff-Nanoröhrchen ("CNT"), Graphen, Nanodrahtgeflechte und/oder ultradünne Metallfilme umfassen, gegebenenfalls wobei die Elektroden ITO umfassen;
(x) die Vorrichtung Einlass-/Auslassöffnungen umfasst, wobei der Abstand zwischen benachbarten Öffnungen im Bereich von etwa 5 mm bis etwa 500 mm liegt; oder
(xi) die auf Elektrobenetzung basierende Vorrichtung mindestens ein Heizelement und mindestens eine Nachweiszone umfasst oder mit diesen in Kontakt ist.

3. Verfahren nach Anspruch 2, Ausführungsform (v), wobei:
die Elektrobenetzungsvorrichtung mit einem Heizelement und einer Nachweiszone in Kontakt ist oder diese umfasst; und wobei
(i) die auf Elektrobenetzung basierende Vorrichtung quadratische Elektroden umfasst, gegebenenfalls wobei die Elektroden etwa 5 mm mal 5 mm groß sind; oder
(ii) die auf Elektrobenetzung basierende Vorrichtung Elektroden umfasst, wobei die Elektroden quadratisch, dreieckig, rechteckig, kreisförmig, trapezförmig und/oder unregelmäßig geformt sind.

## Revendications

1. Procédé d'amplification d'acides nucléiques automatisé qui comprend les étapes suivantes :
(a) la fourniture d'un dispositif basé sur l'électromouillage ;
(b) la fourniture d'au moins deux gouttelettes sur ledit dispositif basé sur l'électromouillage, dans lequel chaque gouttelette comprend des amorces qui s'hybrident à un acide nucléique cible ; et d'un acide nucléique cible ;
(c) l'amplification de l'acide nucléique cible dans chacune desdites gouttelettes en parallèle ;
(d) la quantification de l'acide nucléique cible amplifié dans au moins une gouttelette ; et
(e) après l'obtention d'une quantité souhaitée de l'acide nucléique cible, la récupération d'au moins une gouttelette et l'utilisation de ladite au moins une gouttelette pour l'analyse ou le traitement ultérieur de ladite au moins une gouttelette, **caractérisé en ce que** l'analyse ou le traitement est choisi dans un groupe constitué par le séquençage d'acides nucléiques, le séquençage de nouvelle génération, le séquençage shotgun de génome entier, le séquençage d'exome entier, le séquençage ciblé, le séquençage d'amplicons, le séquençage à extrémités appariées, RIP-seq/CLIP-seq, ChIP-seq, RNA-seq, le séquençage de transcriptomes et methyl-seq.

2. Procédé selon la revendication 1, dans lequel :
(i) lesdites gouttelettes comprennent chacune un acide nucléique cible différent ;
(ii) chacune desdites gouttelettes comprend le même acide nucléique cible ;
(iii) ledit dispositif basé sur l'électromouillage comprend une configuration biplanaire de rangées parallèles d'électrodes pour effectuer des manipulations de gouttelettes faisant intervenir l'électromouillage ;
(iv) lesdites gouttelettes comprennent un mélange de gouttelettes qui contiennent le même acide nucléique cible et des acides nucléiques cibles différents ;
(v) le dispositif basé sur l'électromouillage comprend une configuration planaire d'électrodes qui effectue des manipulations de gouttelettes faisant intervenir l'électromouillage ;
(vi) lesdites gouttelettes comprennent un volume de gouttelettes compris dans la plage d'environ 1 picolitre à environ 5 mL, éventuellement dans lequel lesdites gouttelettes comprennent un volume de gouttelettes d'environ 12,5 mL ;
(vii) le dispositif basé sur l'électromouillage comprend une configuration biplanaire de rangées parallèles d'électrodes ou une configuration planaire d'électrodes pour effectuer des manipulations de gouttelettes faisant intervenir l'électromouillage, et dans lequel lesdites électrodes comprennent des dimensions d'électrode comprises dans la plage d'environ 100 µm par 100 µm à environ 10 cm par 10 cm ;
(viii) le dispositif basé sur l'électromouillage comprend une configuration biplanaire de rangées parallèles d'électrodes ou une configuration planaire d'électrodes pour effectuer des manipulations de gouttelettes faisant intervenir l'électromouillage, et dans lequel lesdites électrodes sont interdigitées ;
(ix) le dispositif basé sur l'électromouillage comprend une configuration biplanaire de rangées parallèles d'électrodes ou une configuration planaire d'électrodes pour effectuer des manipulations de gouttelettes faisant intervenir l'électromouillage, et dans lequel lesdites électrodes comprennent de l'oxyde d'indium et d'étain (« ITO »), des oxydes conducteurs transparents (« TCO »), des polymères conducteurs, des nanotubes de carbone (« CNT »), du graphène, des mailles en nanofils et/ou des films métalliques ultraminces, éventuellement dans lequel lesdites électrodes comprennent de l'ITO ;
(x) ledit dispositif comprend des orifices d'entrée/sortie dans lequel l'espacement entre les orifices adjacents est compris dans la plage d'environ 5 mm à environ 500 mm ; ou
(xi) le dispositif basé sur l'électromouillage comprend ou est en contact avec au moins un élément chauffant et au moins une zone de détection.

3. Procédé selon la revendication 2, mode de réalisation (v), dans lequel :
ledit dispositif d'électromouillage est en contact avec ou comprend un élément chauffant et une zone de détection ; et dans lequel
(i) ledit dispositif basé sur l'électromouillage comprend des électrodes carrées, éventuellement dans lequel lesdites électrodes sont d'environ 5 mm par 5 mm ; ou
(ii) ledit dispositif basé sur l'électromouillage comprend des électrodes, dans lequel lesdites électrodes sont carrées, triangulaires, rectangulaires, circulaires, trapézoïdales et/ou de forme irrégulière.
